# EUROPÄISCHE PATENTSCHRIFT

(11) **EP 1 309 648 B1**
(45) Veröffentlichungstag und Bekanntmachung des Hinweises auf die Patenterteilung: **20.09.2006**
(21) Anmeldenummer: 01971791.7
(22) Anmeldetag: 27.07.2001
(51) Int. Cl.: C08G 77/46, D06M 15/647

(54) **EIN- ODER MEHRFACHQUARTÄRE POLYSILOXANE**
MONO- OR POLY-QUATERNARY POLYSILOXANES
POLYSILOXANES MONO OU POLY-QUATERNAIRES

(30) Priorität: 27.07.2000 DE 10036524
(43) Veröffentlichungstag der Anmeldung: 14.05.2003
(73) Patentinhaber: GE Bayer Silicones GmbH & Co. KG, 40699 Erkrath (DE)
(72) Erfinder: LANGE, Horst, 44879 Bochum (DE); WAGNER, Roland, 53175 Bonn (DE); WITOSSEK, Anita, 40764 Langenfeld (DE); STACHULLA, Karl-Heinz, 51379 Leverkusen (DE); TEUBER, Siegfried, 47799 Krefeld (DE); KROPFGANS, Martin, 51519 Odenthal (DE); SOCKEL, Karl-Heinz, 51373 Leverkusen (DE); FIRSTENBERG, Don, 40699 Erkrath (DE)
(86) Internationale Anmeldenummer: PCT/EP2001/008698
(87) Internationale Veröffentlichungsnummer: WO 2002/010256

(56) Entgegenhaltungen:
- EP-A- 0 282 720
- EP-A- 0 906 930
- EP-A- 0 924 237
- WO-A-00/50491

## Beschreibung

Die Erfindung betrifft ein- oder mehrfachquartäre Polysiloxanen deren Herstellung und Verwendung als Oberflächenausrüstungskomponente.

EP-A-0 441 530 offenbart textile Weichmacher aus Polysiloxane, die die tertiären Aminogruppen in Seitenketten enthalten. Es ist ebenfalls die Reaktion von α,ω-epoxymodifizierten Siloxanen mit Piperazin beschrieben worden, die in Abhängigkeit von der eingesetzten Piperazinmenge zu oligomeren bis polymeren Strukturen mit tertiären Aminofunktionen in der Hauptkette führt, wie in der US 4 847 154 offenbart.

Die zusätzliche Einführung von Ethylenoxid-/Propylenoxideinheiten als hydrophilierender Komponente führt zu einer Verbesserung des Effekts. Hierzu ist einerseits vorgeschlagen worden, in Seitenketten Alkylenoxideinheiten und tertiäre Aminogruppen zu positionieren, die über Esterstrukturen mit der Siloxanhauptkette verbunden sind, wie in der US 5 591 880 und US 5 650 529 beschrieben. Der Nachteil des Konzept ist die komplizierte Veresterung in Gegenwart tertiärer Aminogruppen. Alternativ hierzu ist bekannt, α,ω-epoyxmodifizierte Siloxane mit sekundäre Aminofunktionen aufweisenden Polyalkylenoxiden zur Reaktion zu bringen, wie in der US 5 981 681 beschrieben.

Verzweigte alkylenoxidmodifizierte quarternäre Polysiloxane werden aus α,ω-OH terminierten Polysiloxanen und Trialkoxysilanen mittels Kondensation synthetisiert. US 5 602 224 offenbart quarternäre Ammoniumstrukrturen, in denen über das Silan eingenbracht werden, worin das quarternäre Stickstoffatom durch Alkylenoxideinheiten substituiert ist.

Streng kammartige alkylenoxidmodifizierte Polysiloxanquats sind ebenfalls in der US 5 098 979 beschrieben worden. Die Hydroxylgruppen von kammartig substituierten Polyethersiloxanen werden mit Epichlorhydrin in die entsprechenden Chlorhydrinderivate überführt. Hieran schließt sich eine Quaternierung mit tertiären Aminen an. Nachteilig an dieser Strategie ist der notwendige Umgang mit Epichlorhydrin und die relativ geringe Reaktivität der Chlorhydrin-Gruppierung während der Quaternierung.

Aus diesem Grund heraus sind die Hydroxylgruppen kammartig substituierter Polyethersiloxane alternativ mit Chloressigsäure verestert worden. Durch die Carbonylaktivierung kann die abschließende Quaternierung erleichtert vollzogen werden, wie in der US 5 153 294 und der US 5 166 297 offenbart.

Die nach dem Prioritätstag dieser Anmeldung veröffentlichten WO 01/41719 und WO 01/41720 beschreiben quartäre Polysiloxanverbindungen zur Verwendung in kosmetischen Zusammensetzungen.

α,ω-Diquarternäre Polysiloxane werden in US 4 891 166 beschrieben. Die Synthese erfolgt durch Reaktion von α,ω-Diepoxiden mit tertiären Aminen in Gegenwart von Säuren.

US 4 833 225 offenbart lineare polyquarternäre Polysiloxane, die durch Reaktion von α,ω-Diepoxiden mit di-tertiären Aminen in Gegenwart von Säuren dargestellt werden. Alternativ können α,ω-halogenalkylmodifizierte Siloxane mit di-tertiären Aminen in polymere Polyquats überführt werden, wie in der US 4 587 321 beschrieben.

Die Substanzen gemäß der US 4 891 166, US 4 833 225 und der US 4 587 321 besitzen eine ausgeprägte Tendenz, auf Festkörperoberflächen aufzuziehen. Bei den vorstehend beschriebenen Verbindungen handelt es sich dem Wesen nach entweder um α,ω-difunktionelle Polysiloxane, entsprechende kettenförmige (AB)ₙ Copolymere, kammartig funktionalisierte Siloxane oder aber Produkte mit einem Anteil an Verzweigungsstellen in der Siloxankette.

In der DE-OS 43 18 536, DE-OS 44 37 886 und den Veröffentlichungen von R.Wagner, L.Richter, B.Weiland, J.Reiners, J.Weißmüller, Appl. Organomet. Chem. 10(1996), 437 sowie R.Wagner, L.Richter, Y.Wu, J.Weißmüller, A.Kleewein, E.Hengge, Appl. Organomet. Chem. 12(1998), 265 werden saccharidmodifizierte Siloxanderivate beschrieben, die über zwei voneinander unabhängig bewegliche siliciumhaltige Einheiten verfügen. Hinsichtlich der Eignung als textile Weichmacher oder für die Ausrüstung anderer Oberflächen sind keine Angaben gemacht worden. Es wird weiterhin als nachteilig empfunden, daß der Schritt der Saccharidaddition in den synthetische Prozeß eingeschlossen werden muß.

Es ist deshalb Aufgabe der vorliegenden Erfindung gewesen, Strukturen zur Verfügung zu stellen, die die Nachteile des Standes der Technik nicht aufweisen.

Die Aufgabe wird durch Verbindungen gelöst, die sich aus zwei unabhängig voneinander beweglichen Siloxaneinheiten und einem verbindenden Amin- oder Ammoniumelement zusammengesetzt werden.

Die Aufgabe wird erfindungsgemäß durch ein- oder mehrfachquartäre Polysiloxanderivate der allgemeinen Formel (I) gelöst:

S-K-Q¹-K-S (I)

worin
- S: 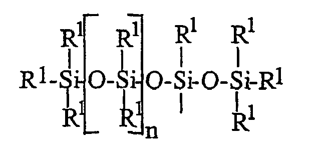
- oder: 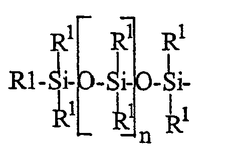
- R¹: C₁-C₂₂-Alkyl, C₁-C₂₂-Fluoralkyl oder Aryl,
- n: 0 bis 1000,
- Q¹: eine sekundäre Aminostruktur 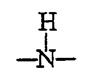 oder tertiäre Aminostruktur 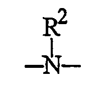 oder quarternäre Ammoniumstruktur 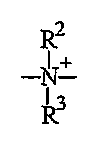
- R²: ein einwertiger oder zweiwertiger geradkettiger, cyclischer oder verzweigter C₁-C₃₀-Kohlenwasserstoffrest, der durch -O-, -NH- -C(O)-, -C(S)- unterbrochen und mit -OH substituiert sein kann oder eine Einfachbindung zu dem Rest K darstellt,
- R³: ein einwertiger geradkettiger, cyclischer oder verzweigter C₁₋C₃₀-Kohlenwasserstoffrest, der durch -O-, -NH-, -C(O)-,-C(S)- unterbrochen und mit -OH substituiert sein kann oder einer Struktur -A-E-, mit
- A: -CH₂C(O)O-, -CH₂CH₂C(O)O- oder -CH₂CH₂CH₂C(O)O- und
- E: einer Polyalkylenoxideinheit der Struktur

-[CH₂CH₂O]_{q}-[CH₂CH(CH₃)O]ᵣ-R⁴
- q: 1 bis 200,
- r: 0 bis 200,
- R⁴: H, geradkettiger, cyclischer oder verzweigter C₁-C₂₀-Kohlenwasserstoffrest, der durch -O- oder -C(O)- unterbrochen und mit -OH substituiert und acetylenisch, olefinisch oder aromatisch sein kann, entspricht,
wobei, wenn eine Mehrzahl von Resten R³ im Molekül vorliegt, diese gleich oder verschieden sein können, sowie
- K: ein zweiwertigen oder dreiwertiger geradkettiger, cyclischer oder verzweigter C₂-C₄₀-Kohlenwasserstoffrest darstellt, der durch -O-, -NH-, -NR¹-, 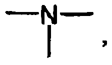 -C(O)-, -C(S)- unterbrochen und mit -OH substituiert sein kann oder eine Einheit Q² enthält, mit
- Q²: sekundäre Aminostruktur 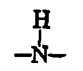 oder tertiäre Aminostruktur 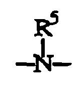 oder quarternäre Ammoniumstruktur 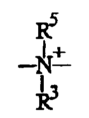
- R⁵: ein einwertiger oder zweiwertiger geradkettiger, cyclischer oder verzweigter C₁-C₂₀-Kohlenwasserstoffrest ist, der durch -O-, -NH-, -C(O)-, -C(S)- unterbrochen und mit -OH substituiert sein kann, wobei die freie Valenz des zweiwertigen Restes R⁵ an Q¹ binden kann,
und wenn eine Mehrzahl von Resten K in den Polysiloxanen vorliegt, diese gleich oder verschieden voneinander sein können,
bedeutet, mit Ausnahme der Verbindung der Formel

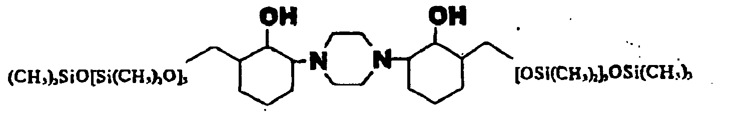

In einer Ausführungsform der Erfindung werden Polysiloxanverbindungen der Formel (I') bereitgestellt:

S-K-Q¹-K-S (I')

besitzen, worin
- S: 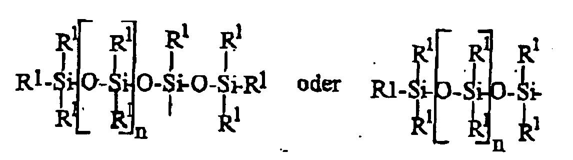
- R¹: C₁-C₂₂-Alkyl, C₁-C₂₂-Fluoralkyl oder Aryl,
- n: 0 bis 1000 bedeuten,

- Q¹: eine sekundäre Aminostruktur 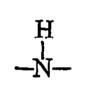 oder tertiäre Aminostruktur 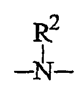 oder quarternäre Ammoniumstruktur 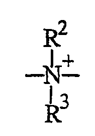
- R² ein: einwertiger oder zweiwertiger geradkettiger, cyclischer oder verzweigten C₁-C₃₀-Kohlenwasserstoffrest, der durch -O-, -NH, -C(O)-, -C(S)- unterbrochen und mit -OH substituiert sein kann oder eine Einfachbindung zu K darstellt, i
- R³: ein einwertiger geradkettiger, cyclischer oder verzweigter C₁₋C₃₀-Kohlenwasserstoffrest, der durch -O-, -NH-, -C(O)-, -C(S)- unterbrochen und mit -OH substituiert sein kann oder einer Struktur -A-E-, mit
- A: -CH₂C(O)O-, -CH₂CH₂C(O)O- oder -CH₂CH₂CH₂C(O)O- und
- E: einer Polyalkylenoxideinheit der Struktur

-[CH₂CH₂O]_{q}-[CH₂CH(CH₃)O]ᵣ-R⁴
- q: 1 bis 200,
- r: 0 bis 200,
- R⁴: H, geradkettiger, cyclischer oder verzweigter C₁-C₂₀-Kohlenwasserstoffrest, der durch -O- oder -C(O)- unterbrochen und mit -OH substituiert und acetylenisch, olefinisch oder aromatisch sein kann, entspricht, sowie
- K: ein zweiwertiger oder dreiwertiger geradkettiger, cyclischer oder verzweigter C₂-C₄₀-Kohlenwasserstofftest, der durch -O-,-NH-, -NR¹-, -N-, -C(O)-, -C(S)- unterbrochen und mit -OH substituiert sein kann oder eine Einheit Q² enthält, mit

- Q²: sekundäre Aminostruktur 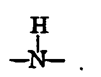 **oder tertiäre Aminostruktur** 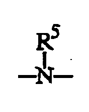 oder quarternäre Ammoniumstruktur 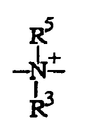
- R⁵: ein einwertiger oder zweiwertiger geradkettiger, cyclischer oder verzweigter C₁-C₂₀-Kohlenwasserstoffrest, der durch-O-, -NH-, -C(O)-, -C(S)- unterbrochen und mit -OH substituiert sein kann oder eine Einfachbindung zu Q¹ darstellt, oder
- R² und R⁵: -CH₃, -CH₂CH₃, -(CH₂)₂CH₃, -(CH₂)₃CH₃, -(CH₂)₅CH₃, -CH₂CH₂OH, 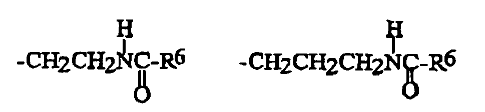
- R⁶: einen geradkettigen, cyclischen oder verzweigten C₁-C₁₈-Kohlenwasserstoffrest, der durch -O-, -NH-, -C(O)-, -C(S)- unterbrochen und mit -OH substituiert sein kann,
bedeutet, mit Ausnahme der verbindung der Formel

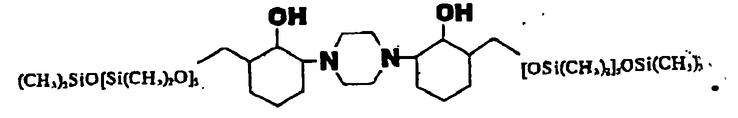

Die Möglichkeit einer dreiwertigen Substruktur für K bedeutet, daß K verzweigt sein kann und somit mit zwei Bindungen an der Quaternierung von Q¹ über den zweiwertigen Rest R² beteiligt ist.

Die Möglichkeit einer zweiwertigen Substruktur für R² bedeutet, daß es sich in diesen Fällen um eine cyclische Systeme bildende Struktur handelt, wobei R² dann eine Einfachbindung zu K, speziell zu einer eine tertiäre Aminostruktur aufweisenden Struktur oder aber zur Quatstruktur Q² über R⁵ ist.

In einer weiteren Ausführungsform der vorliegenden Anmeldung bedeutet R¹ C₁-C₁₈₋Alkyl, C₁-C₁₈-Fluoralkyl und Aryl, und die Reste n, R², R³, R⁴, R⁵, R⁶, K, A, E, Q¹, Q², q und r haben die obengenannte Bedeutung.

In einer weiteren Ausführungsform der vorliegenden Anmeldung bedeutet R¹ C₁-C₁₈₋Alkyl, C₁-C₆-Fluoralkyl und Aryl, und die Reste n, R², R³, R⁴, R⁵, R⁶, K, A, E, Q¹, Q², q und r haben die obengenannte Bedeutung.

In einer weiteren Ausführungsform der vorliegenden Anmeldung bedeutet R¹ C₁-C₆₋Alkyl, C₁-C₄-Fluoralkyl und Phenyl, und die Reste n, R², R³, R⁴, R⁵, R⁶, K, A, E, Q¹, Q², q und r haben die obengenannte Bedeutung.

In einer weiteren Ausführungsform der vorliegenden Anmeldung bedeutet R¹ Methyl, Ethyl, Trfluorpropyl und Phenyl, und die Reste n, R², R³, R⁴, R⁵, R⁶, K, A, E, Q¹, Q², q und r haben die obengenannte Bedeutung.

In einer weiteren Ausführungsform der vorliegenden Anmeldung bedeutet K ein zweiwertiger oder dreiwertiger geradkettiger, cyclischer oder verzweigter C₃-C₃₀₋Kohlenwasserstoffrest, der durch -O-, -NH-, -NR¹-, 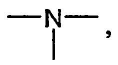 -C(O)-, -C(S)- unterbrochen und mit -OH substituiert sein kann oder eine Einheit Q² enthält, und die Reste n, R¹, R², R³, R⁴, R⁵, R⁶, A, E, Q¹, Q², q und r haben die obengenannte Bedeutung.

In einer weiteren Ausführungsform der vorliegenden Anmeldung, bedeutet n 0 bis 100, bevorzugt 0 bis 80 und besonders bevorzugt 10 bis 80, und die Reste R¹, R², R³, R⁴, R⁵, R⁶, K, A, E, Q¹, Q², q und r haben die obengenannte Bedeutung.

In einer weiteren Ausführungsform der vorliegenden Anmeldung, bedeutet q 1 bis 50, bevorzugt 2 bis 50 und die Reste R¹, R², R³, R⁴, R⁵, R⁶, K, A, E, Q¹, Q², q und r haben die obengenannte Bedeutung.

In einer bevorzugten Ausführungsform der vorliegenden Anmeldung, bedeutet q 2 bis 20 und besonders bevorzugt 2 bis 10 und die Reste R¹, R², R³, R⁴, R⁵, R⁶, K, A, E, Q¹, Q², n und r haben die obengenannte Bedeutung.

In einer weiteren Ausführungsform der vorliegenden Anmeldung, bedeutet r 0 bis 100 und bevorzugt 0 bis 50, und die Reste R¹, R², R³, R⁴, R⁵, R⁶, K, A, E, Q¹, Q², q und n haben die obengenannte Bedeutung.

In einer weiteren bevorzugten Ausführungsform der vorliegenden Anmeldung, bedeutet r 0 bis 20, und besonders bevorzugt 0 bis 10, und die Reste R¹, R², R³, R⁴, R⁵, R⁶, K, A, E, Q¹, Q², q und n haben die obengenannte Bedeutung.

In einer weiteren Ausführungsform der vorliegenden Erfindung bedeutet R² und R⁵⁻CH₃, -CH₂CH₃, -(CH₂)₂CH₃, -(CH₂)₃CH₃, -(CH₂)₅CH₃, -CH₂CH₂OH, 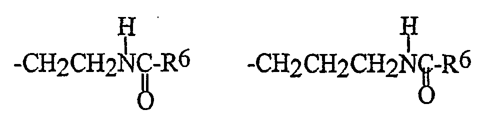 mit R⁶ einen geradkettigen, cyclischen oder verzweigten C₁-C₁₈₋Kohlenwasserstoffrest, der durch -O-, -NH-, -C(O)-, -C(S)- unterbrochen und mit - OH substituiert sein kann,

In einer weiteren Ausführungsform der vorliegenden Erfindung bedeutet R³ -CH₃, -CH₂CH₃, -(CH₂)₂CH₃, -(CH₂)₃CH₃, -(CH₂)₅CH₃, -CH₂CH₂OH, 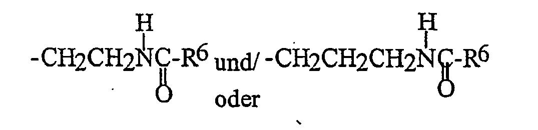 worin R⁶ ein geradkettiger, cyclischer oder verzweigter C₁-C₁₈-Kohlenwasserstoffrest ist, der durch -O-, -NH-, -C(O)-, -C(S)- unterbrochen und mit -OH substituiert sein kann.

In einer weiteren Ausführungsform der vorliegenden Anmeldung bedeutet R⁴ ein zweiwertiger oder dreiwertiger geradkettiger, cyclischer oder verzweigter C₁-C₁₈₋Kohlenwasserstoffrest, der durch -O-, -NH-, -C(O)-, -C(S)- unterbrochen und mit -OH substituiert sein kann oder eine Einfachbindung zu Q¹ enthält, und die Reste n, R¹, R², R³, R⁵, R⁶, K, A, E, Q¹, Q², q und r haben die obengenannte Bedeutung.

In einer weitem bevorzugten Ausführungsform bedeutet R⁴ C₁-C₆-Alkyl, - CH₂CH=CH₂, -CH₂CH(OH)CH₂OCH₂CH=CH₂, -CH₂C≡CH, -C(O)CH₃, - C(O)CH₂CH₃ und die Reste n, R¹, R², R³, R⁵, R⁶, K, A, E, Q¹, Q², q und r haben die obengenannte Bedeutung.

In einer weiteren bevorzugten Ausführungsform der vorleigenden Erfindung bedeutet K 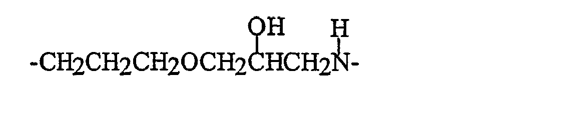 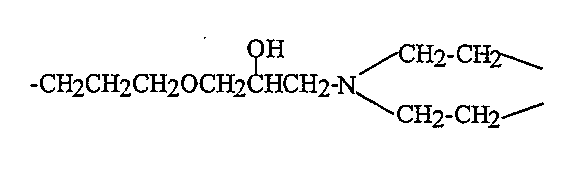 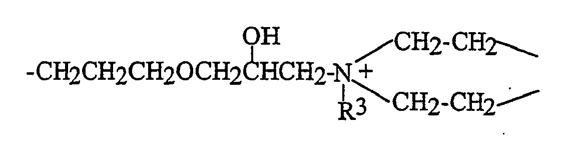 und die Reste n, R¹, R², R³, R⁵, R⁶, A, E, Q¹, Q², q und r haben die obengenannte Bedeutung.

In einer weiteren Ausführungsform der vorliegenden Erfindung bedeutet R⁶ unsubstituierte C₅-C₁₇-Kohlenwasserstoffreste, die sich von den entsprechenden gesättigten oder ungesättigten Fettäuren ableiten, und die Reste n, R¹, R², R³, R⁵, A, E, Q¹, Q², q und r haben die obengenannte Bedeutung.

Der Begriff "C₁-C₂₂-Alkyl oder C₁-C₃₀-Kohlenwasserstoffrest" bedeutet im Rahmen der vorliegenden Erfindung aliphatische Kohlenstoffwasserstoffverbindungen mit 1 bis 22 Kohlenstoffatomen bzw. 1 bis 30 Kohlenstoffatomen die geradkettig oder verzweigt sein können. Beispielhaft seien Methyl, Ethyl, Propyl, n-Butyl, Pentyl, Hexyl, Heptyl, Nonyl, Decyl, Undecyl, iso-Propyl, Neopentyl, und 1,2,3 Trimethylhexyl aufgeführt.

Der Begriff "C₁-C₂₂-Fluoralkyl" bedeutet im Ramen der vorliegenden Erfindung aliphatische Kohlenstoffwasserstoffverbindungen mit 1 bis 22 Kohlenstoffatomen die geradkettig oder verzweigt sein können und mit mindestens einem Fluoratom substituiert sind. Beispielhaft seien Monofluormethyl, Monofluorethyl, 1,1,1-Trifluorethyl, Perflourethyl, 1,1,1-Trifluorpropyl, 1,2,2-Triflourbutyl aufgeführt.

Der Begriff "Aryl " bedeutet im Rahmen der vorliegenden Erfindung unsubstituierte oder ein oder mehrfach mit OH, F, Cl, CF₃ C₁-C₆-Alkyl, C₁-C₆-Alkoxy, C₃-C₇₋Cycloalkyl C₂-C₆-Alkenyl oder Phenyl substituiertes Phenyl. Der Ausdruck kann gegebenenfalls auch Naphthyl bedeuten.

Ein weiterer Gegenstand der vorliegenden Erfindung ist es gewesen ein Verfahren zur Herstellung der Verbindungen der allgemeinen Formel (I) bzw. (I') zur Verfügung zu stellen.

Ausgangspunkt für die Synthesen der erfindungsgemäßen Verbindungen sind monofunktionelle H-Siloxane der allgemeinen Strukturen 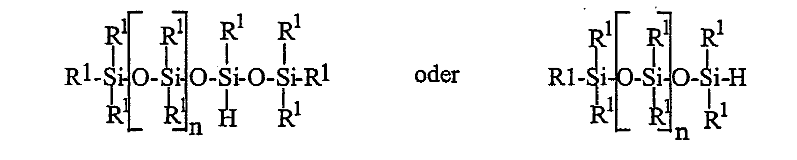 worin R¹ und n die oben angegebenen Bedeutungen haben. Sofern diese Verbindungen nicht kommerziell erhältlich sind, können diese Siloxane, besonders längerkettige Derivate, nach bekannten Verfahren hergestellt werden werden (Silicone, Chemie und Technologie, Vulkan-Verlag, Essen 1989, S. 82-84).

Die sauer katalysierte Äquilibrierung von trimethylsilylterminierten Siloxanen, beispielsweise Hexamethyldisiloxan (MM), mit dimethylsiloxyreichen Verbindungen, beispielsweise Octamethylcyclotetrasiloxan (D₄) in Gegenwart entsprechender Mengen SiH haltiger, aber nicht SiH-endständiger Siloxane liefert Produkte, in denen die SiH-Funktion im Ketteninneren positioniert ist. Im Äquilibrierungsgleichgewicht werden zusätzlich Produkte gebildet, die pro Molekül über keine bzw. mehr als eine SiH-Funktion verfügen.

Die sauer katalysierte Äquilibrierung von α-SiH Verbindungen, von zum Beispiel Pentamethyldisiloxan (MM^{H}) mit dimethylsiloxyreichen Verbindungen, oder beispielsweise Octamethylcyclotetrasiloxan (D₄) liefert monofunktionelle Produkte mit terminaler SiH-Funktion. Das Pentamethyldisiloxan kann zum Beispiel durch äquimolare Mengen Hexamethyldisiloxan (MM) und Tetramethyldisiloxan (M^{H}M^{H}) ersetzt werden. Im Äquilibrierungsgleichgewicht werden zusätzlich Produkte gebildet, die pro Molekül über keine bzw. zwei terminale SiH-Funktion verfügen.

Die Äquilibrierung von cyclischen Siloxanen, wie Hexamethylcyclotrisiloxan (D₃) oder Octamethylcyclotetrasiloxan (D₄) mit Alkalitrimethylsilanolaten, z.B. Kaliumtrimethylsilanolat, liefert Oligosiloxanolate, die mit Dimethylchlorsilan zu den entsprechenden monofunktionellen Verbindungen mit terminaler SiH-Funktion reagieren. Im Äquilibrierungsgleichgewicht werden zusätzlich Produkte gebildet, die pro Molekül über keine bzw. zwei terminale Silanolatfunktionen verfügen. In der Konsequenz liegen ebenfalls Produkte vor, die über keine bzw. zwei terminale SiH-Funktion verfügen.

Im Rahmen der Erfindung werden neben streng definierten monofunktionellen Verbindungen auch Mischungen der vorstehend beschriebenen Art als monofunktionelle SiH-Verbindungen behandelt.

Reaktive, alkylierende, monofunktionelle Siloxanverbindungen werden durch Hydrosilylierung von beispielsweise halogenierten Alkenen, speziell Allylchlorid und Allylbromid, ungesättigten Halogencarbonsäureestern, bevorzugt Chloressigsäureallylester, Chloressigsäurepropargylester, 3-Chlorpropionsäureallylester und 3-Chlorpropionsäurepropargylester und epoxyfunktionellen Alkenen, beispielsweise Vinylcyclohexenoxid und Allylglycidether, mit den vorstehend beschriebenen monofunktionellen SiH-Verbindungen synthetisiert. Die allgemeine Durchführung von Hydrosilylierungen mit Vertretern der genannten Stoffgruppen ist ebenfalls bekannt (B.Marciniec, Comprehensive Handbook on Hydrosilylation, Pergamon Press, Oxford 1992, S. 116-121, 127-130, 134-137, 151-155). Die sich anschließende Synthese sekundäre Aminofunktionen tragender Verbindungen des Typs ABA (ABA meint, das zwei Polysiloxaneinheiten über eine verbrückende Amin- bzw. Ammoniumstruktur verbunden sind) der allgemeinen Struktur

S-K-Q¹-K-S

worin
- Q¹:

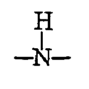

K und S die oben angegebenen Bedeutungen haben, erfolgt bevorzugt durch Alkylierung von zwei primären Aminogruppen aufweisenden Aminen, bespielsweise α,ω-Alkylendiaminen, bevorzugt Ethylendiamin, 1,3-Propylendiamin, 1,6-Hexylendiamin, kurzkettige Ethylenoxid/Propylenoxideinheiten enthaltende diprimäre Amine, speziell Jeffamine® (Huntsman Corp.) der Typen Jeffamin EDR 148, Jeffamin ED 600, Jeffamin D 230, Jeffamin D 400, mit reaktiven, alkylierenden, im Sinne der Erfindung monofunktionellen Siloxanzwischenprodukten. Die Stöchiometrie der Reaktion zwischen dem di-primären Amin zu dem monofunktionelles Siloxan hat ein Verhältnis von 1 : 2.

Die Synthese tertiäre Aminofunktionen tragender Verbindungen des Typs ABA der allgemeinen Struktur

S-K-Q¹-K-S

worin
- Q¹:

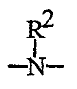

K und S die oben angegebenen Bedeutungen haben, erfolgt bevorzugt auf zwei Wegen. Einerseits ist es möglich, zunächst sekundäre Aminofunktionen tragende ungesättigte Strukturen, beispielsweise N-Methylallylamin oder CH₂=CHCH₂OCH₂CH(OH)CH₂NHCH₃, durch Hydrosilylierung direkt an das monofunktionelle Si-H Siloxan zu binden. Dieser Prozeß ist allgemein bekannt, und zum Beispiel von B.Marciniec, Comprehensive Handbook on Hydrosilylation, Pergamon Press, Oxford 1992, S. 122-124, beschrieben.

Diese gewonnenen sekundären Aminostrukturen können mit den reaktiven, alkylierenden Siloxanzwischenprodukten in einem nachfolgenden Schritt in tertiäre Aminostrukturen tragende Polymere umgewandelt werden. Die Stöchiometrie dieser Reaktion hat ein Verhältnis von Aminosiloxan zu dem monofunktionellen Siloxan von etwa 1 : 1.

Alternativ zu der weiter oben ausgeführten schrittweisen Synthese können die tertiär aminofunktionalisierten Polymeren in einem Reaktionsschritt synthetisiert werden. Ausgangspunkt hierfür sind die behandelten reaktiven, alkylierenden Siloxanzwischenstufen, bevorzugt die Epoxidderivate, speziell die Allylglycidetherspezies. Diese können durch Reaktion mit primären Aminen, beispielsweise Methylamin, in einem molaren Verhältnis von vorzugsweise 2 : 1 in tertiäre Amine überführt werden.

Es können auch difunktionelle sekundäre Amine, zum Beispiel Piperazin, für die Reaktion verwendet werden. Hierbei beträgt das molare Verhältnis von sekundärer Aminogruppe zu der alkylierenden Gruppe, bevorzugt zu einer Epoxygruppe, vorzugsweise 1 : 1. Im Ergebnis einer derartigen Reaktionsführung werden Produkte erhalten, bei denen sich zwei tertiäre Aminogruppen zwischen den beiden Siloxanblöcken befinden.

Die Synthese von ein - oder mehrfachquarternärer Polsiloxanen des Typs ABA der allgemeinen Struktur

S-K-Q¹-K-S

worin
- Q¹: 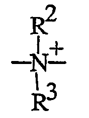
bedeutet, erfolgt ausgehend von tertiären Aminofunktionen enthaltenden Siloxanderivaten auf verschiedenen Wegen. Es ist einerseits bevorzugt, die oben beschriebenen reaktiven, monofunktionellen Siloxanderivate, bevorzugt die epoxyfunktionellen Derivate, mit sekundären Aminen, beispielsweise Dimethylamin oder Morpholin, in tertiäre Amine zu überführen, welche dann in einem nachfolgenden Schritt mit einem zweiten Mol reaktiver, monofunktioneller Siloxanverbindung zu den quarternären Produkten reagieren. Für beide Reaktionsschritte sind molare Verhältnisse von 1: 1 bevorzugt.

Die Verwendung sekundär-tertiärer Diamine eröffnet die Möglichkeit, regioselektiv Kombinationen von tertiären Aminen und quaternären Strukturen zu erzeugen. Die Alkylierung von Aminen des Typs N-Methylpiperazin mit bevorzugt einem Mol epoxyfunktionellem Siloxan liefert di-tertiäre Aminosiloxane, die von einem zweiten Mol reaktiver, monofunktioneller Siloxankomponente, beispielsweise einem Halogencarbonsäureesterderivat, am methylierten Stickstoffatom quaterniert werden. Ein Überschuß an der reaktiven, monofunktionellen Siloxankomponente bzw. der Zuführung eines weiteren Alkylierungsagenz führt zu einer beginnenden Alkylierung des zweiten Stickstoffatoms.

Die durch Alkylierung von sekundären Aminen, beispielsweise Dimethylamin, oder sekundär-tertiären Diaminen, beispielsweise N-Methylpiperazin, mit bevorzugt einem Mol epoxyfunktionellem Siloxan zugänglichen tertiären bzw. di-tertiären Aminosiloxane können in einer bevorzugten Ausführungsform mit difunktionellen Alkylierungsagenzien im molaren Verhältnis 2 : 1 umgesetzt werden. Im Ergebnis einer solchen Reaktion werden zwei quarternäre Ammoniumgruppen oder zwei quarternäre Ammoniumgruppen in Nachbarschaft zu jeweils einer tertiären Aminogruppe über einen einkettigen Spacer miteinander verbunden. Geeignete Alkylierungsmittel für diesen Zweck sind Dihalogenalkane, Diepoxyverbindungen in Gegenwart von Säuren, α,ω-Dihalogenoligoalkylenoxide oder Dihalogencarbonsäureester von Alkylenoxiden.

Bevorzugte Ausgangsmaterialien für α,ω-Dihalogenalkylenoxide und Dihalogencarbonsäureester sind niedermolekulare, oligomere und polymere Alkylenoxide der allgemeinen Zusammensetzung

HO[CH₂CH₂O]_{q}-[CH₂CH(CH₃)O]ᵣH

worin q und r die oben angegebenen Bedeutungen aufweisen. Bevorzugte Vertreter sind Diethylenglycol, Triethylenglycol, Tetraethylenglycol, die Oligoethylenglycole mit Molgewichten von 300 bis 1000 g/mol, bevorzugt 400, 600 und 800, sowie Dipropylenglycol. α,ω-Dihalogenalkylenoxide können in an sich bekannter Weise z.B. durch Halogenierung mit Thionylchlorid erzeugt werden.

Die Versterung erfolgt in an sich bekannter Weise (Organikum, Organisch-chemisches Grundpraktikum, 17. Auflage, VEB Deutscher Verlag der Wissenschaften, Berlin 1988, S. 402-408) durch Reaktion mit den C₂-C₄-Halogencarbonsäuren, deren Anhydriden oder Säurechloriden.

Die vorstehend beschriebenen, bevorzugt aufPiperazin beruhenden, Derivate mit zwei tertiären Aminogruppen zwischen den beiden Siloxanblöcken können ebenfalls in quarternäre Ammoniumsalze überführt werden. Über das molare Verhältnis der beiden tertiären Aminogruppen, die zwischen den zwei Siloxanblöcken eingebunden sind, zu den Alkylierungsagenzien wird der Quaternierungsgrad gesteuert. Es wird bevorzugt, bei äquimolarer Arbeitsweise Produkte zu synthetisieren, in denen alle tertiären Amine in quarternäre Ammoniumfunktionen überführt werden. Andererseits kann es von Vorteil sein, durch einen gezielten Unterschuß an Alkylierungsagenz einen Teil der tertiären Aminofunktionen zu erhalten.

Vorteilhafte Alkylierungsagenzien sind beispielsweise Epoxydrivate in Gegenwart von Säuren, Alkylhalogenide oder Halogencarbonsäureester, bevorzugt Halogencarbonsäureester der Alkylenoxide.

Bevorzugte Ausgangsmaterialien für diese Alkylierungsmittel sind niedermolekulare, oligomere und polymere Alkylenoxide der allgemeinen Zusammensetzung

HO[CH₂CH₂O]_{q}-[CH₂CH(CH₃)O]ᵣR⁴

worin q, r und R⁴ die oben angegebene Bedeutung aufweisen. Bevorzugte Vertreter sind die entsprechend monosubstituierten Derivate von Diethylenglycol, Triethylenglycol, Tetraethylenglycol, der Oligoethylenglycole mit Molgewichten von 300 bis 1000 g/mol, bevorzugt 400, 600 und 800, sowie Dipropylenglycol. Die Herstellung dieser Ether und Ester erfolgt in bekannter Weise durch sauer oder alkalisch katalysierte Addition von Ethylenoxid und/oder Propylenoxid an die entsprechenden Alkohole (Organikum, Organisch-chemisches Grundpraktikum, 17. Auflage, VEB Deutscher Verlag der Wissenschaften, Berlin 1988, S. 259; US 5625024) oder Carbonsäuren (E.Sung, W. Umbach, H. Baumann, Fette Seifen Anstrichmittel 73, 88 [1971]).

Die nachfolgende Synthese der Halogencarbonsäureester erfolgt in an sich bekannter Weise (Organikum, Organisch-chemisches Grundpraktikum, 17. Auflage, VEB Deutscher Verlag der Wissenschaften, Berlin 1988, S. 402-408) durch Reaktion mit den C₂-C₄-Halogencarbonsäuren, deren Anhydriden oder Säurechloriden. Die selektive Synthese hydroxyfunktioneller Halogencarbonsäureester, in denen R⁴ Wasserstoff entspricht, gelingt durch Addition von Ethylenoxid und/oder Propylenoxid an die entsprechenden Halogencarbonsäuren unter sauren Bedingungen.

Mit der Einführung von mehr als einer tertiären Aminofunktion zwischen den Siloxanblöcken, z.B. durch Piperazinstrukturen, ergibt sich in der Folge die Möglichkeit, die Hydrophilie und das Aufziehverhalten auf Oberflächen in erweiterten Grenzen durch das Verhältnis des tertiären Amins zu der quaternären Struktur einzustellen. Es liegt im Rahmen der Erfindung, mehrere Siloxankomponenten und/oder Alkylierungsagenzien unter Beibehaltung der gewünschten Gesamtstöchiometrie zur Reaktion zu bringen. Es folgt hieraus z.B.die Möglichkeit, eine gewünschte Siloxankettenlänge durch Einsatz einer einzigen Siloxankomponente oder aber durch gezielte Mischung mehrerer Siloxankomponenten einzustellen.

Als Anionen kommen bevorzugt die während der Quaternierung gebildeten Halogenidionen, speziell Chloridionen, in Betracht. Durch Ionenaustauschreaktionen können auch andere Anionen verwendet werden. Beispielhaft seien organische Anionen, wie Carboxylate, Sulfonate, Sulfate, Polyethercarboxylate und Polyethersulfate aufgeführt.

Die Alkylierungsreaktionen werden bevorzugt in polaren organischen Lösungsmitteln ausgeführt. Geeignet sind zum Beispiel Alkohole aus der Gruppe bestehend aus Methanol, Ethanol, i-Propanol und n-Butanol; Glycole aus der Gruppe bestehend aus Ethylenglycol, Diethylenglycol, Triethylenglycol, die Methyl-, Ethyl- und Butylether der genannten Glycole, 1,2-Propylenglycol und 1,3-Propylenglycol, Ketone, wie Aceton und Methylethylketon, Ester, wie Ethylacetat, Butylacetat und 2-Ethylhexylacetat, Ether, wie Tetrahydrofuran und Nitroverbindungen, wie Nitromethan. Die Wahl des Lösungsmittels richtet sich im Wesentlichen nach der Löslichkeit der Reaktionspartner und der angestrebten Reaktionstemperatur. Die Reaktionen werden im Bereich von 20 °C bis 130 °C, vorzugsweise 40 °C bis 100 °C ausgeführt.

Die erfindungsgemäßen Produkte, welche in sich die weichmachenden Eigenschaften von Siloxanstrukturen und die Tendenz von Aminostrukturen bzw. quarternären Ammoniumgruppen zur Adsorption an negativ geladenen Festkörperoberflächen vereinen, können mit Erfolg eingesetzt werden in kosmetischen Formulierungen für die Haut- und Haarpflege, in Polituren für die Behandlung und Ausrüstung harter Oberflächen, in Formulierungen zum Trocknen von Automobilen und anderen harten Oberflächen nach maschinellen Wäschen, zur Ausrüstung von Textilen und Textilfasern, als separate Weichmacher nach dem Waschen von Textilien mit nichtionogenen oder anionischen/nichtionogen Detergenzienformulierungen, als Weichmacher in auf nichtionischen oder anionischen/nichtionischen Tensiden beruhenden Formulierungen zur Textilwäsche.

Aminoderivate können hierbei in Abhängigkeit vom pH-Wert in Form der Amine oder Aminsalze eingesetzt werden.

Die Erfindung betrifft des weiteren die Verwendung der vorstehend beschriebenen Polysiloxanverbindungen in kosmetischen Formulierungen für die Haut- und Haarpflege, in Polituren für die Behandlung und Ausrüstung harter Oberflächen, in Formulierungen zum Trocknen von Automobilen und anderen harten Oberflächen, zum Beispiel nach maschinellen Wäschen, zur Ausrüstung von Textilien und Textilfasern, als separate Weichmacher nach dem Waschen von Textilien mit nichtionogenen oder anionischen/nichtionogen Detergenzienformulierungen, als Weichmacher in auf nichtionischen oder anionischen/nichtionischen Tensiden beruhenden Formulierungen zur Textilwäsche, sowie als Mittel zur Verhinderung bzw. Rückgängigmachung von Textilverknitterungen.

Die Erfindung betrifft des weiteren die Verwendung der vorstehend beschriebenen Polysiloxanverbindungen als waschbeständige hydrophile Weichmacher für die textile Erstausrüstung.

Ferner betrifft die Erfindung Zusammensetzungen, die mindestens eine der Polysiloxanverbindungen zusammen mit mindestens einem weiteren für die Zusammensetzung üblichen Inhaltsstoff enthält.

Im folgenden sind einige typische Beispiele für derartige Zusammensetzungen gegeben, in denen die Polysiloxanverbindungen der Erfindung vorteilhaft verwendet werden können.

Typische Hilfsstoffe in derartigen Zusammensetzungen sind z.B. diejenigen Stoffe , die in A. Domsch: Die kosmetischen Präparate Bd. I u. II 4. Aufl. Verl. für chem. Industrie, H. Ziolkowsky KG , Augsburg sowie International Cosmetic Ingredient Dictionary and Handbook 7^{th} Ed. 1997 by J.A. Wenniger, G.N. McEwen Vol. 1-4 by The Cosmetic, Toiletry and Fragrance Association Washington DC bzw. unter http://www.cosmetic-world.com/inci /Incialf.htm beschrieben sind.

### Anionisches Shampoo

Das Formulierungsbeispiel ist als Rahmenrezeptur gedacht. Anionisches Shampoo enthalten üblicherweise die folgenden Komponenten, ohne auf diese beschränkt zu sein:
Alkylsulfate, Alkylethersulfate, Natriumlaurylsulfat, Natriumlauryl-ethersulfat, Ammoniumlaurylsulfat, Ammoniumlauryl-ethersulfat, TEA-laurylsulfat, TEA-laurylethersulfat, Alkylbenzolsulfonate, α-Olefinsulfonate, Paraffinsulfonate, Sulfosuccinate, N-Acyltauride, Sulfat-glyceride, Sulfatierte Alkanolamide, Carboxylatsalze, N-acyl-Aminosäuresalze, Silicone, etc.

| Komponente | % |
|---|---|
| Ammoniumlaurylsulfat | 10.00 - 30.00 |
| Ammoniumlauryl-ethersulfat | 5.00 - 20.00 |
| Cocamidopropyl Betaine | 0.00 - 15.00 |
| Lauramid DEA | 0.00 - 5.00 |
| Cocamid Mea | 0.00 - 5.00 |
| Dimethicone Copolyol (Dimethylsiloxanglykolcopolymer) | 0.00 - 5.00 |
| Cyclopentasiloxane | 0.00 - 5.00 |
| Erfindungsgemäße Polysiloxanverbindung | 0.50 - 5.00 |
| Polyquaternium-10 | 0.00 - 2.00 |
| Konservierungsmittel | 0.00 - 0.50 |
| Duftstoffe | 0.00 - 5.00 |
| Entionisiertes Wasser | q.s. 100% |
| Natriumchlorid | q.s. |

### Nichtionisches Shampoo

Das Formulierungsbeispiel ist als Rahmenrezeptur gedacht. Nichtionische Shampoos enthalten üblicherweise die folgenden Komponenten, ohne auf diese beschränkt zu sein:
Monoalkanolamide, Monoethanolamide, Monoisopropanolamide, Polyhydroxyderivative, Sucrosemonolaurat, Polyglycerinether, Aminoxide, Polyethoxylierte Derivative, Sorbitanderivative, Silicone, etc.

| Komponente | % |
|---|---|
| Lauramid DEA | 10.00 - 30.00 |
| Lauramid-Oxid | 5.00 - 20.00 |
| Cocamid Mea | 0.00 - 5.00 |
| Dimethicone Copolyol | 0.00 - 5.00 |
| Erfindungsgemäße Polysiloxanverbindung | 0.50 - 5.00 |
| Konservierungsmittel | 0.00 - 0.50 |
| Duftstoffe | 0.00 - 5.00 |
| Entionisiertes Wasser | q.s. 100% |
| Natriumchlorid | q.s. |

### Amphoteres Shampoo

Das Formulierungsbeispiel ist als Rahmenrezeptur gedacht. Formulierungen dieser Kategorie enthalten üblicherweise die folgenden Komponenten, ohne auf diese beschränkt zu sein:
N-Alkyl-iminodipropionate, N-Alkyl-iminopropionate, Aminosäuren, Aminosäurederivative, Amidobetaine, Imidazoliniumderivative, Sulfobetaine, Sultaine, Betaine, Silicone etc.

| Komponente | % |
|---|---|
| PEG-80-sorbitanlaurat | 10.00 - 30.00 |
| Lauroamphoglycinat | 0.00 - 10.00 |
| Cocamidopropyl-Hydroxysultain | 0.00 - 15.00 |
| PEG-150-distearat | 0.00 - 5.00 |
| Laurylether-13-carboxylat | 0.00 - 5.00 |
| Erfindungsgemäße Polysiloxanverbindung | 0.50 - 5.00 |
| Duftstoffe | 0.00 - 5.00 |
| Entionisiertes Wasser | q.s. 100% |
| Natriumchlorid | q.s. |

### Kationisches Shampoo

Das Formulierungsbeispiel ist nur als Rahmenrezeptur gedacht. Formulierungen dieser Kategorie enthalten die folgenden Komponenten, ohne auf diese beschränkt zu sein:
Bis-Quartäre Ammoniumverbindungen, Bis-(trialkylammoniumacetyl)diamine, Amidoamine, Ammonioalkylester, Silicone etc.

| Komponente | % |
|---|---|
| Laurylether-13-carboxylat | 10.00 - 30.00 |
| Isopropylmyristat | 5.00 - 20.00 |
| Cocamidopropyl-Betaine | 0.00 - 15.00 |
| Lauramid DEA | 0.00 - 5.00 |
| Cocamid MEA | 0.00 - 5.00 |
| Erfindungsgemäße Polysiloxanverbindung | 0.50 - 5.00 |
| Konservierungsmittel | 0.00 - 0.50 |
| Duftstoffe | 0.00 - 5.00 |
| Entionisiertes Wasser | q.s. 100% |
| Natriumchlorid | q.s. |

### Festiger

Das Formulierungsbeispiel ist nur als Rahmenrezeptur gedacht. Formulierungen dieser Kategorie enthalten üblicherweise die folgenden Komponenten, ohne auf diese beschränkt zu sein:
Fettsäuren, Fettsäureester, Ethoxylierte Fettsäuren, Ethoxylierte Fettsäureester, Fettalkohole, Ethoxylierte Fettalkohole, Glycole, Glycolester, Glycerin, Glycerinester, Lanolin, Lanolinderivative, Mineralöl, Petrolatum, Lecithin, Lecithinderivative, Wachse, Wachsderivative, Kationische Polymere, Proteine, Proteinderivative, Aminosäuren, Aminosäurederivative, Feuchthaltemittel, Verdickungsmittel, Silicone etc.

| Komponente | % |
|---|---|
| Ceteareth-20 | 0.10 - 10.00 |
| Steareth-20 | 0.10 - 10.00 |
| Stearyl-Alkohol | 0.10 - 10.00 |
| Stearamidopropyl-Dimethylamin | 0.00 - 10.00 |
| Dicetyldimonium-Chlorid | 0.00 - 10.00 |
| Erfindungsgemäße Polysiloxanverbindung | 0.50 - 5.00 |
| Cyclopentasiloxan | 0.00 - 5.00 |
| Dimethicone | 0.00 - 5.00 |
| Konservierungsmittel | 0.00 - 0.50 |
| Duftstoffe | 0.00 - 5.00 |
| Entionisiertes Wasser | q.s. 100% |

### "Clear Rinse -Off"-Festiger

Das Formulierungsbeispiel ist als Rahmenrezeptur gedacht. Formulierungen dieser Kategorie enthalten üblicherweise die folgenden Komponenten, ohne auf diese beschränkt zu sein:
Fettsäuren, Fettsäureester, Ethoxylierte Fettsäuren, Ethoxylierte Fettsäureester, Fettalkohole, Ethoxylierte Fettalkohole, Glycole, Glycolester, Glycerin, Glycerinester, Lanolin, Lanolinderivative, Mineralöl, Petrolatum, Lecithin, Lecithinderivative, Wachse, Wachsderivative, Kationische Polymere, Proteine, Proteinderivative, Aminosäuren, Aminosäurederivative, Feuchthaltemittel, Verdickungsmittel, Silicone etc.

| Komponente | % |
|---|---|
| Glycerin | 0.10 - 10.00 |
| Cetrimonium-Chlorid | 0.00 - 10.00 |
| Erfindungsgemäße Polysiloxanverbindung | 0.50 - 5.00 |
| Hydroxyethylcellulose | 0.00 - 5.00 |
| Konservierungsmittel | 0.00 - 0.50 |
| Duftstoffe | 0.00 - 5.00 |
| Entionisiertes Wasser | q.s. 100% |

### Schaumfestiger für Haare

Das Formulierungsbeispiel ist als Rahmenrezeptur gedacht. Formulierungen dieser Kategorie enthalten die folgenden Komponenten, ohne auf diese beschränkt zu sein:
Fettsäuren, Fettsäureester, Ethoxylierte Fettsäuren, Ethoxylierte Fettsäureester, Fettalkohole, Ethoxylierte Fettalkohole, Glycole, Glycolester, Glycerin, Glycerinester, Lanolin, Lanolinderivative, Mineralöl, Petrolatum, Lecithin, Lecithinderivative, Wachse, Wachsderivative, Kationische Polymere, Proteine, Proteinderivative, Aminosäuren, Aminosäurederivative, Feuchthaltemittel, Verdickungsmittel, Silicone Lösungsmittel, Ethanol, Isopropanol, Isoparaffinlösungsmittel, Butan, Propan, Isobutan, CFCs, Fluorierte Aereosoltreibmittel, Dimethylether, komprimierte Gase, etc.

| Komponente | % |
|---|---|
| Erfindungsgemäße Polysiloxanverbindung | 0.50 - 5.00 |
| Nonoxynol-15 | 0.00 - 2.00 |
| Nonoxynol-20 | 0.00 - 2.00 |
| Duftstoffe | 0.00 - 5.00 |
| Aereosoltreibmittel | 0.00 - 20.00 |
| Konservierungsmittel | 0.00 - 0.50 |
| Entionisiertes Wasser | q.s. 100% |

### Pumpspray (Festiger) für Haare

Das Formulierungsbeispiel ist nur als Rahmenrezeptur gedacht. Formulierungen dieser Kategorie enthalten üblicherweise die folgenden Komponenten, ohne auf diese beschränkt zu sein:
Fettsäuren, Fettsäureester, Ethoxylierte Fettsäuren, Ethoxylierte Fettsäureester, Fettalkohole, Ethoxylierte Fettalkohole, Glycole, Glycolester, Glycerin, Glycerinester, Lanolin, Lanolinderivative, Mineralöl, Petrolatum, Lecithin, Lecithinderivative, Wachse, Wachsderivative, Kationische Polymere, Proteine, Proteinderivative, Aminosäuren, Aminosäurederivative, Feuchthaltemittel, Verdickungsmittel, Silicone Lösungsmittel, Ethanol, Isopropanol, Isoparaffinlösungsmittel, etc.

| Komponente | % |
|---|---|
| Erfindungsgemäße Polysiloxanverbindung | 0.50 - 5.00 |
| Cyclomethicone | 0.00 - 80.00 |
| Ethanol | 0.00 - 80.00 |
| Konservierungsmittel | 0.00 - 0.50 |
| Duftstoffe | 0.00 - 5.00 |
| Entionisiertes Wasser | q.s. 100% |

### Festigerspray für Haare

Das Formulierungsbeispiel ist als Rahmenrezeptur gedacht. Formulierungen dieser Kategorie enthalten üblicherweise die folgenden Komponenten, ohne auf diese beschränkt zu sein:
Fettsäuren, Fettsäureester, Ethoxylierte Fettsäuren, Ethoxylierte Fettsäureester, Fettalkohole, Ethoxylierte Fettalkohole, Glycole, Glycolester, Glycerin, Glycerinester, Lanolin, Lanolinderivative, Mineralöl, Petrolatum, Lecithin, Lecithinderivative, Wachse, Wachsderivative, Kationische Polymere, Proteine, Proteinderivative, Aminosäuren, Aminosäurederivative, Feuchthaltemittel, Verdickungsmittel, Silicone Lösungsmittel, Ethanol, Isopropanol, Isoparaffinlösungsmittel, Butan, Propan, Isobutan, CFCs, Fluorierte Aereosoltreibmittel, Dimethylether, Komprimierte Gase, etc.

| Komponente | % |
|---|---|
| Erfindungsgemäße Polysiloxanverbindung | 0.50 - 5.00 |
| Cyclomethicone | 0.00 - 80.00 |
| Ethanol | 0.00 - 50.00 |
| Aereosoltreibmittel | 0.00 - 50.00 |
| Konservierungsmittel | 0.00 - 0.50 |
| Duftstoffe | 0.00 - 5.00 |
| Entionisiertes Wasser | q.s. 100% |

### Gelfestiger für Haare

Das Formulierungsbeispiel ist als Rahmenrezeptur gedacht. Formulierungen dieser Kategorie enthalten üblichwerweise die folgenden Komponenten, ohne auf diese beschränkt zu sein:
Verdickungsmittel, Cellulosederivative, Acrylsäurederivative, Fixativ-Polymere, Konditionierungschemikalien, Glykole, Glykolester, Glycerin , Glycerinester, Lanolin, Lanolinderivative, Mineralöl, Petrolatum, Lecithin, Lecithinderivative, Wachse, Wachsderivative, Kationische Polymere, Proteine, Proteinderivative, Aminosäuren, Aminosäurederivative, Feuchthaltemittel, Silicone, Lösungsmittel, Ethanol, Isopropanol, Isoparaffin-Lösungsmittel etc.

| Komponente | % |
|---|---|
| Erfindungsgemäße Polysiloxanverbindung | 0.50 - 5.00 |
| Hydroxyethylcellulose | 0.00 - 2.00 |
| Duftstoffe | 0.00 - 5.00 |
| Konservierungsmittel | 0.00 - 0.50 |
| Zitronensäure | 0.00 - 2.00 |
| Entionisiertes Wasser | q.s. 100% |

### Styling Gel für Haare

Das Formulierungsbeispiel ist als Rahmenrezeptur gedacht. Formulierungen dieser Kategorie enthalten üblicherweise die folgenden Komponenten, ohne auf diese beschränkt zu sein:
Fixativ-Polymere, Lacke, Acrylisäurederivative, Cellulosederivative, Vinylderivative, Konditionierungschemikalien, Glykole, Glykolester, Glycerin , Glycerinester, Lanolin, Lanolinderivative, Mineralöl, Petrolatum, Lecithin, Lecithinderivative, Wachse, Wachsderivative, Kationische Polymere, Proteine, Proteinderivative, Aminosäuren, Aminosäurederivative, Feuchthaltemittel, Verdickungsmittel, Silicone, Lösungsmittel, Ethanol, Isopropanol, Isoparaffin-Lösungsmittel etc.

| Komponente | % |
|---|---|
| Erfindungsgemäße Polysiloxanverbindung | 0.50 - 5.00 |
| Fixative | 0.10-10.00 |
| Hydroxyethylcellulose | 0.00 - 2.00 |
| Duftstoffe | 0.00 - 5.00 |
| Zitronensäure | 0.00 - 2.00 |
| Entionisiertes Wasser | q.s. 100% |

### Styling Spray für Haare

Das Formulierungsbeispiel ist als Rahmenrezeptur gedacht. Formulierungen dieser Kategorie enthalten üblicherweise die folgenden Komponenten, ohne auf diese beschränkt zu sein:
Fixativ-Polymere, Lacke, Vinylderivative, Fettsäuren, Fettsäureester, Ethoxylierte Fettsäuren, Ethoxylierte Fettsäureester, Fettalkohole, Ethoxylierte Fettalkohole, Glykole, Glykolester, Glycerin , Glycerin-Ester, Lanolin, Lanolinderivative, Mineral öl, Petrolatum, Lecithin; Lecithinderivative, Wachse, Wachsderivative, Kationische Polymere, Proteine, Proteinderivative, Aminosäuren, Aminosäurederivative, Feuchthaltemittel, Verdickungsmittel, Silicone, Lösungsmittel, Ethanol, Isopropanol, Isoparaffinlösungsmittel, Butan, Propan, Isobutan, CFCs, Fluorierte Aerosoltreibmittel, Dimethylether, Komprimierte Gase, etc.

| Komponente | % |
|---|---|
| Erfindungsgemäße Polysiloxanverbindung | 0.50 - 5.00 |
| Cyclomethicone | 0.00 - 80.00 |
| Fixative | 0.10-10.00 |
| Ethanol | 0.00 - 50.00 |
| Aerosoltreibmittel | 0.00 - 50.00 |
| Konservierungsmittel | 0.00 - 0.50 |
| Duftstoffe | 0.00 - 5.00 |
| Entionisiertes Wasser | q.s. 100% |

### Pumpspray (Styling) für Haare

Das Formulierungsbeispiel ist als Rahmenrezeptur gedacht. Formulierungen dieser Kategorie enthalten üblicherweise die folgenden Komponenten, ohne auf diese beschränkt zu sein:
Vinylderivative, Fixativ-Polymere, Lacke, Fettsäuren, Fettsäureester, Ethoxylierte Fettsäuren, Ethoxylierte Fettsäureester, Fettalkohole, Ethoxylierte Fettalkohol, Glykole, Glykolester, Glycerin , Glycerinester, Lanolin, Lanolinderivative, Mineralöl, Petrolatum, Lecithin, Lecithinderivative, Wachse, Wachsderivative, Kationische Polymere, Proteine, Proteinderivative, Aminosäuren, Aminosäurederivative, Feuchthaltemittel, Verdickungsmittel, Silicone, Lösungsmittel, Ethanol, Isopropanol, Isoparaffinlösungsmittel, Butan, Propan, Isobutan, CFCs, Fluorierte Aerosoltreibmittel, Dimethylether, komprimierte Gase, etc.

| Komponente | % |
|---|---|
| Erfindungsgemäße Polysiloxanverbindung | 0.50 - 5.00 |
| Fixative | 0.10-10.00 |
| Cyclomethicone | 0.00 - 80.00 |
| Ethanol | 0.00 - 50.00 |
| Konservierungsmittel | 0.00 - 0.50 |
| Duftstoffe | 0.00 - 5.00 |
| Entionisiertes Wasser | q.s. 100% |

Die Verwendung der erfindungsgemäßen Polysiloxanderivate führt bei Anwendung im Haarkosmetikbereich zu günstigen Effekten hinsichtlich Festigung, Glanz, Fixierung (Halt), Körper, Volumen, Feuchtigkeitsregulierung, Farbretention, Schutz vor Umwelteinflüssen (UV, Salzwasser u.s.w.), Wiederformbarkeit, Antistatischen Eigenschaften, Färbbarkeit etc.

### Beispiele

Die nachfolgenden Beispiele dienen der näheren Erläuterung der vorliegenden Erfindung, ohne sie jedoch einzuschränken

### Beispiel 1

1a) 33,7 g (0,1 mol) eines Epoxysiloxans der Formel 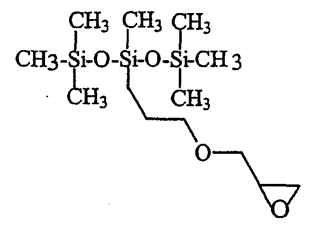 und 10,1g (0,1 mol) N-Methylpiperazin wurden in 40 ml i-Propanol gelöst und 7 Stunden auf Rückflußtemperatur erhitzt. Das Lösemittel destillierte man nach dem Reaktionsende im Wasserstrahl- und anschließend im Ölpumpenvakuum ab. Es wurden 39 g einer klaren, hellbraunen Flüssigkeit der Struktur 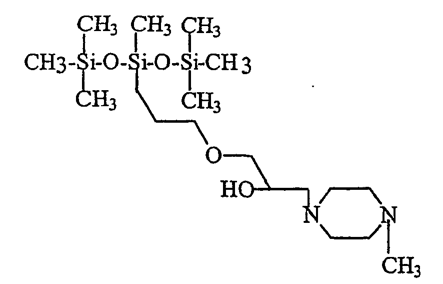 erhalten. Gemäß einer gaschromatographischen Analyse wurde das Epoxid quantitativ in das Piperazinderivat überführt.
1b) 497 g (8,87 mol) CH CCH₂OH wurden unter Stickstoff bei Raumtemperatur vorgelegt. Unter intensiven Rühren wurden innerhalb 1 Stunde 955 g (8,45 mol) Chloressigsäurechlorid zugetropft. Während des Zutropfens stieg die Temperatur auf 60 °C an und eine intensive HCl-Entwicklung setzte ein. Der Ansatz färbte sich schwarz. Nach Beendigung des Zutropfens wurde der Ansatz 1 Stunde auf 130 °C erhitzt. Eine fraktionierte Destillation ergab als Hauptlauf 891g eines leicht gelblichen Öls der Struktur CH CCH₂OC(O)CH₂Cl mit einem Siedepunkt von 179-181 °C. Die gaschromatographisch bestimmte Reinheit des Esters betrug 99%.

| ¹³C-NMR: | |
|---|---|
| **Substruktur** | **shift (ppm)** |
| ClCH₂C(O)OCH₂C CH | 40,4 |
| ClCH₂C(O)OCH₂C CH | 166,5 |
| ClCH₂C(O)OCH₂C CH | 53,1 |
| ClCH₂C(O)OCH₂C CH | 76,4 |
| ClCH₂C(O)OCH₂C CH | 75,6 |

1c) 26,5 g (0,2 mol) des Chloressigsäureesters gemäß Beispiel 1b und 44 mg einer 3,43 % Pt enthaltenden Lamoreaux-Katalysatorlösung gemäß US 3 220 972 wurden bei Raumtemperatur unter Stickstoff vorgelegt. Innerhalb 30 Minuten wurden 48,8 g (0,22 mol) 1,1,1,3,5,5,5-Heptamethyltrisiloxan (M₂D^{H}) zugetropft und die Temperatur auf 60 °C erhöht. Im Anschluß wurde der Ansatz 4 Stunden auf 100 °C erhitzt. Nach dem Abdestillieren aller bis 120 °C und bei 2hPa siedenden Bestandteile wurden 64,5 g einer gelblichen Flüssigkeit erhalten. Gemäß einer gaschromatographischen Analyse enthielt das Produkt zu 85 % das Zielprodukt 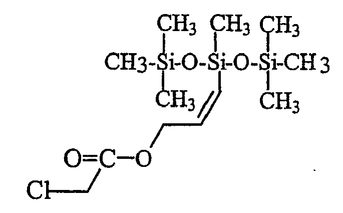 und 15 % des Heptamethyltrisiloxanylesters der Chloressigsäure.

| ¹³C-NMR des Si-C verknüpften Zielproduktes | |
|---|---|
| **Substruktur** | **shift (ppm)** |
| ClCH₂C(O)OCH₂CH=CH-Si | 40,3 |
| ClCH₂C(O)OCH₂CH=CH-Si | 166,7 |
| ClCH₂C(O)OCH₂CH=CH-Si | 67,8 |
| ClCH₂C(O)OCH₂CH=CH-Si | 144,4 |
| ClCH₂C(O)OCH₂CH=CH-Si | 126,6 |

1d) 21,8 g (0,05 mol) des siloxanylmodifizierten Piperazinderivates gemäß Beispiel 1a) und 17,7 g (0,05 mol) des Chloressigsäureesterderivates gemäß Beispiel 1c) wurden unter Stickstoff in 50 ml Methylpropylketon aufgenommen und 6 Stunden auf Rückflußtemperatur erhitzt. Nach Beendigung der Reaktion wurden alle bis 100 °C und bei 4 hPa siedenden Bestandteile im Vakuum entfernt. Es wurden 35,7 g einer zähen, braunen Masse der Struktur 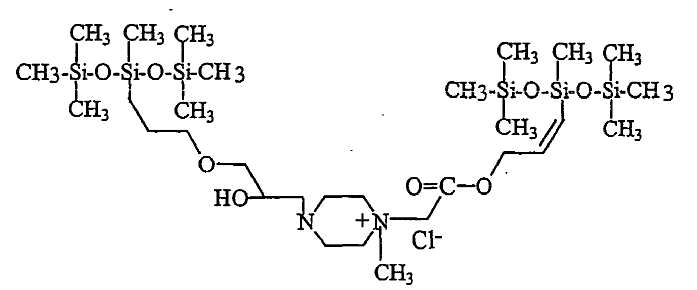 erhalten.

| ¹³C-NMR des Si-C verknüpften Zielproduktes | |
|---|---|
| **Substruktur** | **shift (ppm)** |
| -CH(OH)CH₂NCH₂CH₂N⁺(CH₃)CH₂C(O)OCH₂CH=CH-Si | 65,7 |
| -CH(OH)CH₂NCH₂CH₂N⁺(CH₃)CH₂C(O)OCH₂CH=CH-Si | 51,2 |
| -CH(OH)CH₂NCH₂CH₂N⁺(CH₃)CH₂C(O)OCH₂CH=CH-Si | 46,4 |
| -CH(OH)CH₂NCH₂CH₂N⁺(CH₃)CH₂C(O)OCH₂CH=CH-Si | 60,3 |
| -CH(OH)CH₂NCH₂CH₂N⁺(CH₃)CH₂C(O)OCH₂CH=CH-Si | 52,8 |
| -CH(OH)CH₂NCH₂CH₂N⁺(CH₃)CH₂C(O)OCH₂CH=CH-Si | 61,0 |
| -CH(OH)CH₂NCH₂CH₂N⁺(CH₃)CH₂C(O)OCH₂CH=CH-Si | 169,0 |
| -CH(OH)CH₂NCH₂CH₂N⁺(CH₃)CH₂C(O)OCH₂CH=CH-Si | 66,5 |
| -CH(OH)CH₂NCH₂CH₂N⁺(CH₃)CH₂C(O)OCH₂CH=CH-Si | 144,1 |
| -CH(OH)CH₂NCH₂CH₂H⁺(CH₃)CH₂C(O)OCH₂CH=CH-Si | 126,0 |

### Beispiel 2

2a) 238 g (2,24 mol) Diethylenglycol wurden unter Stickstoff bei Raumtemperatur vorgelegt. Unter intensiven Rühren tropfte man innerhalb einer Stunde 558 g (4,93 mol) Chloressigsäurechlorid zu. Während des Zutropfens stieg die Temperatur auf 82 °C an und eine starke HCl-Entwicklung setzte ein. Nach Beendigung des Zutropfens wurde der Ansatz 30 Minuten auf 130 °C erhitzt. Abschließend wurden alle bis 130 °C und bei 20 hPa siedenden Bestandteile abdestilliert. Man erhielt 566g eines hellgelben Öls der Zusammensetzung

ClCH₂C(O)OCH₂CH₂OCH₂CH₂OC(O)CH₂Cl

Die gaschromatographisch bestimmte Reinheit des Esters betrug 99,2 %.

| ¹³C-NMR: | |
|---|---|
| **Substruktur** | **shift (ppm)** |
| ClCH₂- | 40,7 |
| ClCH₂-C(O)- | 167,1 |
| ClCH₂-C(O)-OCH₂- | 65,2 |
| ClCH₂-C(O)-OCH₂CH₂- | 68,6 |

2b) 21,8 g (0,05 mol) der siloxanylmodifizierten Piperazinverbindung gemäß Beispiel 1a) und 6,46 g (0,025 mol) des Chloressigsäureesterderivates gemäß Beispiel 2a) wurden unter Stickstoff in 100 ml i-Propanol gelöst und 10 Stunden auf Rückflußtemperatur erhitzt. Nach Beendigung der Reaktion wurden alle Bestandteile die bis 70 °C und bei 20 hPa siedeten entfernt. Man erhielt 26,1 g einer harten, amorphen, gelb-braunen Masse der Formel

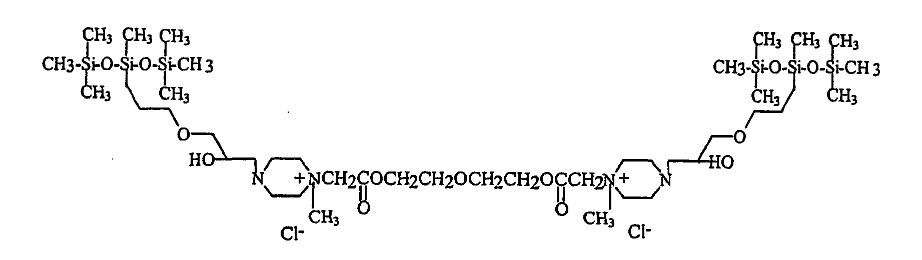

(Die Verbindung entspricht folgender Definition des Anspruchs:
R¹ = Methyl
n = 0
K (linksseitig) = 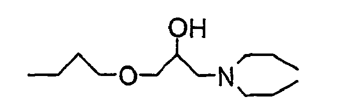
Q1= 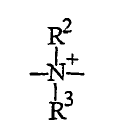
Mit R3 = Methyl und R2= Bindung an K
K (rechtsseitig) =
Q2<K'
Mit Q2 = 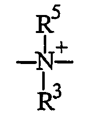
Mit R3 = Methyl
Und R5 = -CH₂-CO-O-CH₂CH₂OCH₂CH₂O-CO-CH₂-
K' =

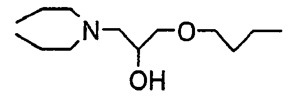

| ¹³C-NMR | |
|---|---|
| **Substruktur** | **shift (ppm)** |
| -CH(OH)-CH₂-N-CH₂-CH₂-N⁺-CH₂-C(O)- | 66,0 |
| -CH(OH)-CH₂-N-CH₂-CH₂-N⁺-CH₂-C(O)- | 52,5 |
| -CH(OH)-CH₂-N-CH₂-CH₂-N⁺-CH₂-C(O)- | 45,6 |
| -CH(OH)-CH₂-N-CH₂-CH₂-N⁺-CH₂-C(O)- | 60,4 |
| -CH(OH)-CH₂-N-CH₂-CH₂-N⁺-CH₂-C(O)- | 61,3 |
| -CH(OH)-CH₂-N-CH₂-CH₂-N⁺-CH₂-C(O)- | 169,2/169,8 |
| CH₃-N⁺ | 52,9 |

### Beispiel 3

110 g (0,03 mol) eines epoxymodifizierten Siloxans der statistischen Zusammensetzung 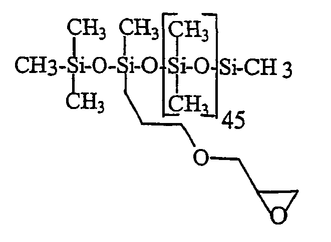 und 1,3 g (0,015 mol) Piperazin wurden in 120 ml i-Propanol aufgenommen und 5 Stunden auf Rückflußtemperatur erhitzt. Nach Beendigung der Reaktion wurden alle bis 100 °C und bei 4 hPa flüchtigen Bestandteile im Vakuum entfernt. Es wurden 109,7 g eines hellgelben Öls der Struktur 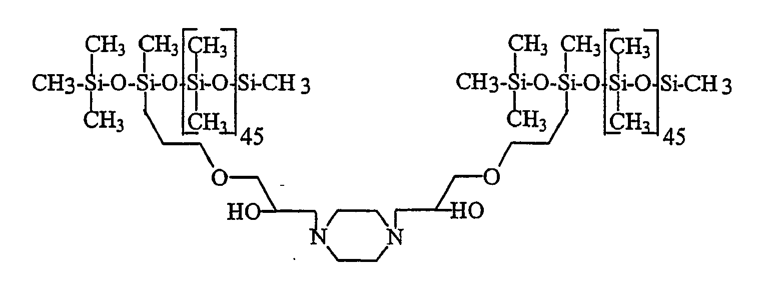 erhalten.

| ¹³C-NMR | |
|---|---|
| **Substruktur** | **shift (ppm)** |
| -CH(OH)CH₂NCH₂ | 66,0 |
| -CH(OH)CH₂NCH₂ | 60,5 |
| -CH(OH)CH₂NCH₂ | 53,2 |

### Beispiel 4

Zum Nachweis der weichmachenden Eigenschaften als interner Weichmacher während des Waschprozesses wurden gebleichte und an der Oberfläche nicht weiter ausgerüstete Baumwollstreifen einem Waschprozeß in Gegenwart von Ariel Futur® , bentonithaltigem Dash 2 in 1® sowie des in Beispiel 2 beschriebenen Aminosiloxans unterworfen. Es wurden folgende Randbedingungen eingehalten.

| | Streifen 1 | Streifen 2 | Streifen 3 |
|---|---|---|---|
| Streifengewicht (g) | 13,40 | 13,55 | 13,29 |
| Wassermenge (ml) | 669 | 679 | 665 |
| Detergenz | 0,66g Ariel Futur® | 0,68g Ariel Futur® | 0,64g Dash 2 in 1® |
| Siloxan Bsp. 2 | 0,2 g | - | - |
| Note 0 | 1,5 | 2,8 | 1,7 |

Das Wasser wurde auf 60 °C erhitzt, die Detergenzien und im Falle des Baumwollstreifens 1 zusätzlich das Aminosiloxan gemäß Beispiel 2 gelöst. Anschließend wurden die Baumwollstreifen in diesen Lösungen für 30 Minuten gewaschen. Nachfolgend wurden die Streifen fünfmal mit jeweils 600 ml Wasser gespült und abschließend 30 Minuten bei 120°C getrocknet.

14 Testpersonen bewerteten die drei Baumwollstreifen auf die Weichheit des Griffs hin, wobei die Note 1 dem weichesten Streifen und die Note 3 dem als am härtesten empfundenen Streifen zugeteilt wurde.

Im Ergebnis der Bewertung erhielt der Baumwollstreifen 1 die Durchschnittsnote 1,5. Der Baumwollstreifen 2 wurde durchschnittlich mit 2,8 und der bentonitbehandelte Streifen 3 mit 1,7 bewertet.

## Patentansprüche

1. Ein- oder mehrfachquarternäre Polysiloxane der allgemeinen Formel (I),
S-K-Q¹-K-S (I)
worin
S 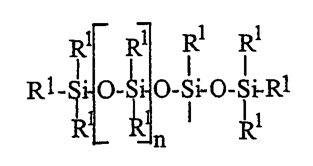
oder 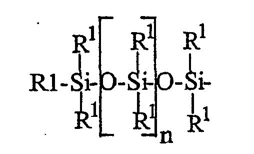
R¹ C₁-C₂₂-Alkyl, C₁-C₂₂-Fluoralkyl oder Aryl,
n 0 bis 1000,
Q¹ eine sekundäre Aminostruktur 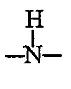 oder tertiäre Aminostruktur 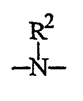 oder quarternäre Ammoniumstruktur 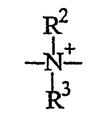
R² ein einwertiger oder zweiwertiger geradkettiger, cyclischer oder verzweigter C₁-C₃₀-Kohlenwasserstoffrest, der durch -O-, -NH- -C(O)-, -C(S)- unterbrochen und mit -OH substituiert sein kann oder eine Einfachbindung zu dem Rest K darstellt,
R³ ein einwertiger geradkettiger, cyclischer oder verzweigter C₁₋C₃₀-Kohlenwasserstoffrest, der durch -O-, -NH-, -C(O)-, -C(S)- unterbrochen und mit -OH substituiert sein kann oder einer Struktur -A-E-, mit
A -CH₂C(O)O-, -CH₂CH₂C(O)O- oder -CH₂CH₂CH₂C(O)O- und
E einer Polyalkylenoxideinheit der Struktur
-[CH₂CH₂O]_{q}-[CH₂CH(CH₃)O]ᵣ-R⁴
q 1 bis 200,
r 0 bis 200,
R⁴ H, geradkettiger, cyclischer oder verzweigter C₁-C₂₀-Kohlenwasserstoffrest, der durch -O- oder -C(O)- unterbrochen und mit -OH substituiert und acetylenisch, olefinisch oder aromatisch sein kann, entspricht,
wobei, wenn eine Mehrzahl von Resten R³ im Molekül vorliegt, diese gleich oder verschieden sein können, sowie
K ein zweiwertigen oder dreiwertiger geradkettiger, cyclischer oder verzweigter C₂-C₄₀-Kohlenwasserstoffrest darstellt, der durch -O-,-NH-, -NR¹-, 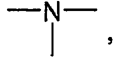 -C(O), -C(S)- unterbrochen und mit -OH substituiert sein kann oder eine Einheit Q² enthält, mit
Q² sekundäre Aminostruktur 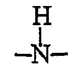 oder tertiäre Aminostruktur 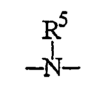 oder quarternäre Ammoniumstruktur 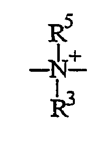
R⁵ ein einwertiger oder zweiwertiger geradkettiger, cyclischer oder verweigter C₁-C₂₀-Kohlenwasserstoffrest ist, der durch -O-, -NH-, -C(O)-, -C(S)- unterbrochen und mit -OH substituiert sein kann, wobei die freie Valenz des zweiwertigen Restes R⁵ an Q¹ binden kann,
und wenn eine Mehrzahl von Resten K in den Polysiloxanen vorliegt, diese gleich oder verschieden voneinander sein können,
bedeutet, mit Ausnahme der Verbindung der Formel

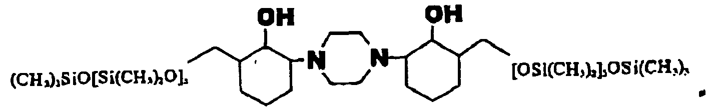

2. Ein- oder mehrfachquarternäre Polysiloxane gemäß Anspruch 1, **dadurch gekennzeichnet, daß** n 0 bis 100, bevorzugt 0 bis 80 und besonders bevorzugt 10 bis 80 ist.

3. Ein- oder mehrfachquarternäre Polysiloxane gemäß einem der vorhergehenden Ansprüche, **dadurch gekennzeichnet, daß** q 1 bis 50 ist.

4. Ein- oder mehrfachquarternäre Polysiloxane gemäß einem der vorhergehenden Ansprüche, **dadurch gekennzeichnet, daß** r 0 bis 100 und bevorzugt 0 bis 50 ist.

5. Ein- oder mehrfachquarternäre Polysiloxane gemäß einem der vorhergehenden Ansprüche, **dadurch gekennzeichnet, daß** R² und R⁵ -CH₃, -CH₂CH₃, -(CH₂)₂CH₃, -(CH₂)₃CH₃, -(CH₂)₅CH₃, -CH₂CH₂OH, 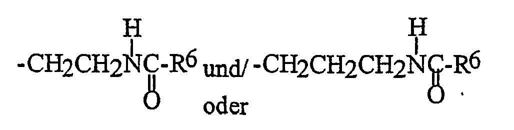 sind, worin R⁶ ein geradkettiger, cyclischer oder verzweigter C₁-C₁₈₋Kohlenwasserstoffrest ist, der durch -O-, -NH-, -C(O)-, -C(S)- unterbrochen und mit -OH substituiert sein kann.

6. Ein- oder mehrfachquarternäre Polysiloxane gemäß einem der vorhergehenden Ansprüche, **dadurch gekennzeichnet, daß** R³ -CH₃, -CH₂CH₃, -(CH₂)₂CH₃, -(CH₂)₃CH₃, -(CH₂)₅CH₃, -CH₂CH₂OH, 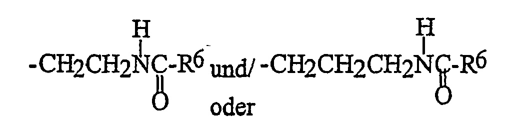 ist, worin R⁶ ein geradkettiger, cyclischer oder verzweigter C₁-C₁₈₋Kohlenwasserstoffrest ist, der durch -O-, -NH-, -C(O)-, -C(S)- unterbrochen und mit -OH substituiert sein kann.

7. Ein- oder mehrfachquarternäre Polysiloxane gemäß einem der vorhergehenden Ansprüche, **dadurch gekennzeichnet, dass** K einen zweiwertigen oder dreiwertigen geradkettigen, cyclischen oder verzweigten C₃-C₃₀₋Kohlenwasserstoffrest darstellt, der durch -O-, -NH-, NR¹-, 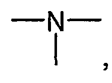 -C(O)-, -C(S)- unterbrochen und mit -OH substituiert sein kann und/oder eine Einheit Q² enthält.

8. Verfahren zur Herstellung von ein- oder mehrfachquarternären Polysiloxanen gemäß irgend einem der Ansprüche 1 bis 7, **dadurch gekennzeichnet, daß** zur Herstellung quarternäre Ammoniumgruppen enthaltender Verbindungen der allgemeinen Struktur
S-K-Q¹-K-S
worin
Q¹ 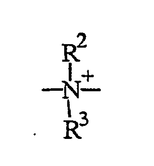
ist, tertiäre Aminofunktionen enthaltende, monofunktionelle Siloxanderivate mit reaktiven, monofunktionellen Siloxanderivaten alkyliert werden, die durch Hydrosilylierung von beispielsweise halogenierten Alkenen, ungesättigten Halogencarbonsäureestern, und epoxy-funktionellen Alkenen, mit monofunktionellen SiH-Verbindungen der allgemeinen Strukturen 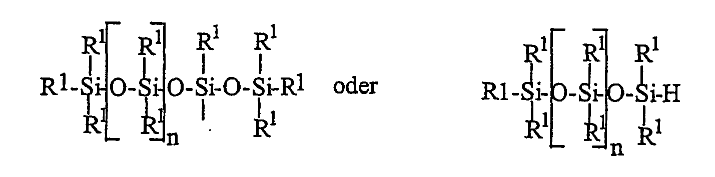 synthetisiert werden, worin das molare Verhältnis der tertiären Aminofunktionen zu den reaktiven, alkylierenden Gruppen zweckmäßig 100: 1 bis 1 : 1 ist.

9. Verfahren zur Herstellung von ein- oder mehrfachquarternären Polysiloxanen nach Anspruch 8, **dadurch gekennzeichnet, daß** als halogenierte Alkene bevorzugt Allylchlorid und Allylbromid verwendet werden.

10. Verfahren zur Herstellung von ein- oder mehrfachquarternären Polysiloxanen gemäß Anspruch 8, **dadurch gekennzeichnet, daß** als ungesättigten Halogencarbonsäureestern bevorzugt solche aus der Gruppe bestehend aus Chloressigsäureallylester, Chloressigsäurepropargylester, 3-Chlorpropionsäureallylester und 3-Chlorpropionsäurepropargylester verwendet werden.

11. Verfahren zur Herstellung von ein- oder mehrfachquarternären Polysiloxanen gemäß Anspruch 8, **dadurch gekennzeichnet, daß** als epoxy-funktionellen Alkenen bevorzugt Vinylcyclohexenoxid und Allylglycidether verwendet werden.

12. Verfahren zur Herstellung von ein- oder mehrfachquarternären Polysiloxanengemäß irgend einem der Ansprüche 1 bis 7, **dadurch gekennzeichnet, daß** zur Herstellung tertiäre Aminofunktionen tragender Verbindungen der allgemeinen Struktur
S-K-Q¹-K-S
worin
Q¹ 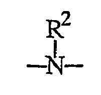
ist, und K und S die Bedeutungen gemäß Anspruch 1 haben, sekundäre Aminofunktionen tragende ungesättigte Strukturen, durch Hydrosilylierung direkt an das monofunktionelle Si-H-Siloxan gebunden werden und nachfolgend mit monofunktionellen, reaktiven, alkylierenden Siloxanzwischenprodukten in tertiäre Aminostrukturen tragende Verbindungen umgewandelt werden, worin die Stöchiometrie des sekundären Amins zu dem reaktiven, alkylierenden Siloxan zweckmäßig 1 : 1 beträgt.

13. Verfahren zur Herstellung von ein- oder mehrfachquarternären Polysiloxanen gemäß Anspruch 12, **dadurch gekennzeichnet, daß** als sekundäre Aminofunktionen tragende ungesättigte Strukturen, bevorzugt N-Methylallylamin oder CH₂=CHCH₂OCH₂CH(OH)CH₂NHCH₃ verwendet werden.

14. Verfahren zur Herstellung von ein- oder mehrfachquarternären Polysiloxanen gemäß irgend einem der Ansprüche 1 bis 7, **dadurch gekennzeichnet, daß** zur Herstellung tertiäre Aminofunktionen tragender Verbindungen der allgemeinen Struktur
S-K-Q¹-K-S
worin
Q¹ 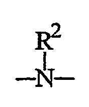 ist und K und S die Bedeutung gemäß Anspruch 1 haben, di-sekundäre Amine, bevorzugt Piperazin, mit monofunktionellen, reaktiven, alkylierenden Siloxanzwischenprodukten in tertiäre Aminostrukturen tragende Verbindungen umgewandelt werden, wobei die Stöchiometrie des di-sekundären Amins zu dem reaktiven, alkylierenden Siloxan zweckmäßig etwa 1 : 2 beträgt.

15. Verfahren zur Herstellung von ein- oder mehrfachquarternären Polysiloxanen gemäß irgend einem der Ansprüche 1 bis 7, **dadurch gekennzeichnet, daß** zur Herstellung von äquimolare Mengen an tertiären Aminofunktionen und quarternären Ammoniumgruppen enthaltenden Verbindungen der allgemeinen Struktur
S-K-Q¹-K-S
sekundär-tertiäre Diamine, bevorzugt N-Methylpiperazin, mit monofunktionellen, reaktiven, alkylierenden Siloxanzwischenprodukten, bevorzugt Epoxyderivaten, in di-tertiäre Aminosiloxanstrukturen tragende Verbindungen umgewandelt werden, wobei die Stöchiometrie des sekundär-tertiären Diamins zu dem monofunktionellen, reaktiven, alkylierenden Siloxan zweckmäßig etwa 1 : 1 beträgt, und nachfolgend die di-tertiären Aminosiloxansbuktwen mit einem Mol einer monofunktionellen, reaktiven, alkylierenden Siloxanverbindung zu den tertiäre Aminogruppen und quarternäre Ammoniumgruppen enthaltenden Siloxanderivaten umgesetzt werden.

16. Verfahren zur Herstellung von ein- oder mehrfachquarternären Polysiloxanen gemäß irgend einem der Ansprüche 1 bis 15, **dadurch gekennzeichnet, daß** zur Herstellung tertiäre Aminofunktionen und quarternäre Ammoniumgruppen tragender Verbindungen der allgemeinen Struktur
S-K-Q¹-K-S
di-sekundäre Amine, bevorzugt Piperazin, mit monofunktionellen, reaktiven, alkylierenden Siloxanzwischenprodukten in tertiäre Aminostrukturen tragende Verbindungen umgewandelt werden, worin die Stöchiometrie des di-sekundären Amins zu dem reaktiven, alkylierenden Siloxan 1 : 2 beträgt und nachfolgend eine Alkylierung, mit Epoxiden in Gegenwart von Säuren, Alkylhalogeniden oder Halogencarbonsäureestern, bevorzugt mit Halogencarbonsäureestern der Alkylenoxide, erfolgt, worin das molare Verhältnis der tertiären Aminogruppen zu den Alkylierungsagenzien zweckmäßig 100 : 1 bis 1: 1 beträgt.

17. Verfahren zur Herstellung von Polysiloxanderivaten gemäß irgend einem der Ansprüchen 1 bis 16, **dadurch gekennzeichnet, daß** zur Herstellung von quarternäre Ammoniumgruppen und tertiären Aminofunktionen enthaltenden Verbindungen der allgemeinen Struktur
S-K-Q¹-K-S
sekundäre Amine, bevorzugt Dimethylamin, oder sekundär-tertiäre Diamine, bevorzugt N-Methylpiperazin, mit monofunktionellen, reaktiven, alkylierenden Siloxanzwischenprodukten, bevorzugt Epoxyderivaten, in tertiäre oder di-tertiäre Aminosiloxanstrukturen tragende Verbindungen umgewandelt werden, wobei die Stöchiometrie des sekundärem Amins oder des sekundär-tertiären Diamins zu dem monofunktionellen, reaktiven, alkylierenden Siloxan zweckmäßig etwa 1 : 1 beträgt, und nachfolgend die gebildeten tertiären bzw. di-tertiären Aminosiloxanstrukturen mit einem difunktionellen Alkylierungsagenz bevorzugt im molaren Verhältnis 2 : 1 zu quarternären Ammoniumgruppen oder quarternären Ammoniumgruppen und gleichzeitig tertiäre Aminostrukturen enthaltenden Siloxanderivaten umgesetzt werden.

18. Verfahren zur Herstellung von Siloxanderivaten vom gemäß irgend einem der Ansprüche 8 bis 17, **dadurch gekennzeichnet, daß** die Halogencarbonsäureester auf niedermolekularen, oligomeren und polymeren Alkylenoxiden der allgemeinen Zusammensetzung
HO[CH₂CH₂O]_{q}-[CH₂CH(CH₃)O]ᵣR⁴
worin q, r und R⁴ die Bedeutungen gemäß Anspruch 1 aufweisen, und bevorzugt monosubstituierten Derivate aus der Gruppe bestehend aus Diethylenglycol, Triethylenglycol, Tetraethylenglycol, oder Oligoethylenglycole mit Molgewichten von 300 bis 1000g/mol, und Dipropylenglycol verwendet werden.

19. Verfahren zur Herstellung von Siloxanderivaten gemäß einem der Ansprüche 8 bis 18, **dadurch gekennzeichnet, daß** als Halogencarbonsäureester auf niedermolekularen, oligomeren und polymeren Alkylenoxiden bevorzugt solche aus der Gruppe der Oligoethylenglycole mit Molgewichten von 400, 600 und 800 g/mol verwendet werden.

20. Verwendung von ein- oder mehrfach quarternären Polysiloxanen, in denen zwei Siloxaneinheiten über Amino- oder Ammoniumeinheiten miteinander verbunden sind, gemäß Ansprüchen 1 bis 7 oder die nach einem der Ansprüche 8 bis 19 erhältlich sind, in kosmetischen Formulierungen für die Haut- und Haarpflege, in Polituren für die Behandlung und Ausrüstung harter Oberflächen, in Formulierungen zum Trocknen von Automobilen und anderen harten Oberflächen nach maschinellen Wäschen, zur Ausrüstung von Textilen und Textilfasern, als separate Weichmacher nach dem Waschen von Textilien mit nichtionogenen oder anionischen/nicht-ionogen Detergenzienformulierungen, als Weichmacher in auf nichtionischen oder anionischen/nichtionischen Tensiden beruhenden Formulierungen zur Textilwäsche, wobei Aminogruppen in Abhängigkeit vom pH-Wert in Form der Amine oder Aminsalze eingesetzt werden.

## Claims

1. Mono- or poly-quaternary polysiloxanes of the general formula (I),
S-K-Q¹-K-S (I)
in which
S 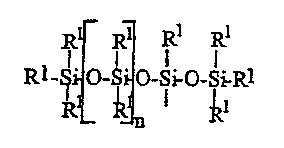
or 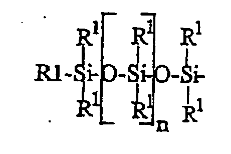
R₁ is C₁-C₂₂-alkyl, C₁-C₂₂-fluoroalkyl or aryl,
n is 0 to 1000,
Q¹ is a secondary amino structure 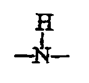 or tertiary amino structure 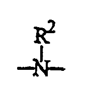 or quaternary ammonium structure 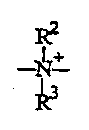
R² is a monovalent or divalent straight-chain, cyclic or branched C₁-C₃₀-hydrocarbon radical which can be interrupted by -O-, -NH-, -C(O)-, -C(S)- and be substituted by -OH or is a single bond to the radical K,
R³ is a monovalent straight-chain, cyclic or branched C₁-C₃₀-hydrocarbon radical which may be interrupted by -O-, -NH-, -C(O)-, -C (S) - and be substituted by -OH, or is a structure -A-E-, where
A is -CH₂C(O)O-, -CH₂CH₂C(O)O- or -CH₂CH₂CH₂C(O)O- and
E is a polyalkylene oxide unit of the structure
-[CH₂CH₂O]_{q}-[CH₂CH(CH₃)O]ᵣ-R⁴
q is 1 to 200,
r is 0 to 200,
R⁴ is H, straight-chain, cyclic or branched C₁-C₂₀-hydrocarbon radical which may be interrupted by -O- or -C(O)- and substituted by -OH, and be acetylenic, olefinic or aromatic,
where, if a plurality of radicals R³ is present in the molecule, these may be identical or different, and
K is a divalent or trivalent straight-chain, cyclic or branched C₂-C₄₀-hydrocarbon radical which may be interrupted by -O-, -NH-, -NR¹-, 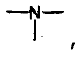 -C(O)-, -C (S) - and substituted by -OH, or contains a unit Q² where
Q² is a secondary amino structure 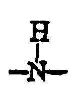 or tertiary amino structure 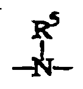 or quaternary ammonium structure 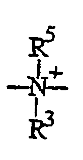
R⁵ is a monovalent or divalent straight-chain, cyclic or branched C₁-C₂₀-hydrocarbon radical which may be interrupted by -O-, -NH-, -C(O)-, -C(S)- and be substituted by -OH, where the free valency of the divalent radical R⁵ can bind to Q¹,
and if a plurality of radicals K is present in the polysiloxanes, these may be identical or different from one another,
with the exception of the compound of the formula

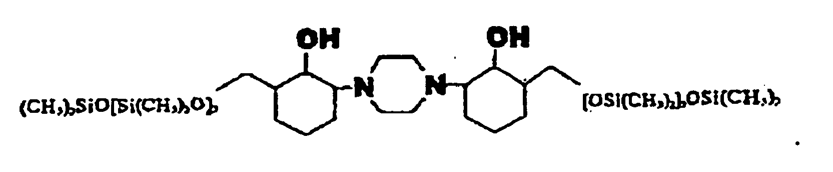

2. Mono- or poly-quaternary polysiloxanes according to Claim 1, **characterized in that** n is 0 to 100, preferably 0 to 80 and particularly preferably 10 to 80.

3. Mono- or poly-quaternary polysiloxanes according to one of the preceding claims, **characterized in that** q is 1 to 50.

4. Mono- or poly-quaternary polysiloxanes according to one of the preceding claims, **characterized in that** r is 0 to 100 and preferably 0 to 50.

5. Mono- or poly-quaternary polysiloxanes according to one of the preceding claims, **characterized in that** R² and R⁵ are -CH₃, -CH₂CH₃, -(CH₂)₂CH₃, -(CH₂)₃CH₃, -(CH₂)₅CH₃, -CH₂CH₂OH, 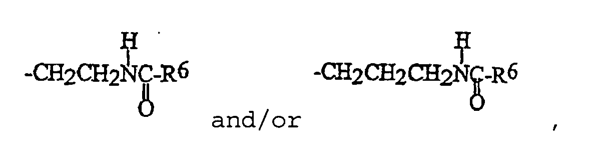 in which R⁶ is a straight-chain, cyclic or branched C₁-C₁₈-hydrocarbon radical which may be interrupted by -O-, -NH-, -C(O)-, -C(S)- and substituted by -OH.

6. Mono- or poly-quaternary polysiloxanes according to one of the preceding claims, **characterized in that** R³ is -CH₃, -CH₂CH₃, -(CH₂)₂CH₃, - (CH₂)₃CH₃, - (CH₂)₅CH₃, -CH₂CH₂OH, 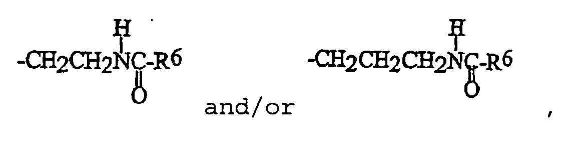 in which R⁶ is a straight-chain, cyclic or branched C₁-C₁₈-hydrocarbon radical which may be interrupted by -O-, -NH-, -C(O)-, -C(S)- and substituted by -OH.

7. Mono- or poly-quaternary polysiloxanes according to one of the preceding claims, **characterized in that** K is a divalent or trivalent straight-chain, cyclic or branched C₃-C₃₀-hydrocarbon radical which may be interrupted by -O-, -NH-, -NR¹-, 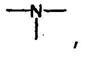 -C(O)-, -C(S)- and substituted by -OH, and/or contains a unit Q².

8. Process for the preparation of mono- or poly-quaternary polysiloxanes according to any one of Claims 1 to 7, **characterized in that**, for the preparation of compounds containing quaternary ammonium groups and of the general structure
S-K-Q¹-K-S
in which
Q¹ is 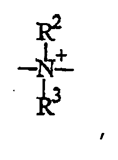
monofunctional siloxane derivatives containing tertiary amino functions are alkylated with reactive, monofunctional siloxane derivatives, which are synthesized by hydrosilylation of, for example, halogenated alkenes, unsaturated halocarboxylic acid esters, and epoxy-functional alkenes, with monofunctional SiH compounds of the general structures 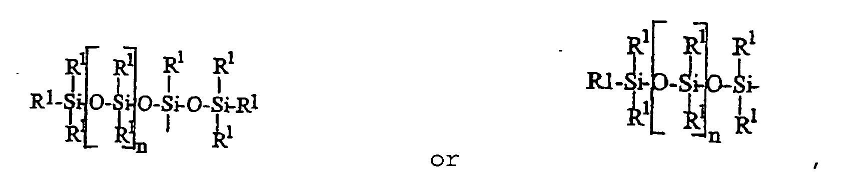 in which the molar ratio of the tertiary amino functions to the reactive, alkylating groups is expediently 100:1 to 1:1.

9. Process for the preparation of mono- or poly-quaternary polysiloxanes according to Claim 8, **characterized in that** the halogenated alkenes used are preferably allyl chloride and allyl bromide.

10. Process for the preparation of mono- or poly-quaternary polysiloxanes according to Claim 8, **characterized in that** the unsaturated halocarboxylic acid esters used are preferably those from the group consisting of allyl chloroacetate, propargyl chloroacetate, allyl 3-chloropropionate and propargyl 3-chloropropionate.

11. Process for the preparation of mono- or poly-quaternary polysiloxanes according to Claim 8, **characterized in that** the epoxy-functional alkenes used are preferably vinylcyclohexene oxide and allyl glycidyl ether.

12. Process for the preparation of mono- or poly-quaternary polysiloxanes according to any one of Claims 1 to 7, **characterized in that**, for the preparation of compounds carrying tertiary amino functions and of the general structure
S-K-Q¹-K-S
in which
Q¹ is 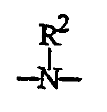
and K and S have the meanings according to Claim 1, unsaturated structures carrying secondary amino functions are bonded directly to the monofunctional Si-H siloxane by hydrosilylation and are subsequently converted to compounds carrying tertiary amino structures with monofunctional, reactive, alkylating siloxane intermediates, in which the stoichiometry of the secondary amine to the reactive, alkylating siloxane is expediently 1:1.

13. Process for the preparation of mono- or poly-quaternary polysiloxanes according to Claim 12, **characterized in that** the unsaturated structures carrying secondary amino functions used are preferably N-methylallylamine or CH₂=CHCH₂OCH₂CH(OH)CH₂NHCH₃.

14. Process for the preparation of mono- or poly-quaternary polysiloxanes according to any one of Claims 1 to 7, **characterized in that**, for the preparation of compounds carrying tertiary amino functions and of the general structure
S-K-Q¹-K-S
in which
Q¹ is 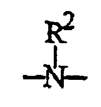
and K and S have the meaning according to Claim 1, di-secondary amines, preferably piperazine, are converted to compounds carrying tertiary amino structures with monofunctional, reactive, alkylating siloxane intermediates, where the stoichiometry of the di-secondary amine to the reactive, alkylating siloxane is expediently about 1:2.

15. Process for the preparation of mono- or poly-quaternary polysiloxanes according to any one of Claims 1 to 7, **characterized in that**, for the preparation of compounds containing equimolar amounts of tertiary amino functions and quaternary ammonium groups and of the general structure
S-K-Q¹-K-S
secondary-tertiary diamines, preferably N-methylpiperazine, are converted into compounds carrying di-tertiary aminosiloxane structures with monofunctional, reactive, alkylating siloxane intermediates, where the stoichiometry of the secondary-tertiary diamine to the monofunctional, reactive, alkylating siloxane is expediently about 1:1, and subsequently the di-tertiary aminosiloxane structures are reacted with one mol of a monofunctional, reactive, alkylating siloxane compound to give the siloxane derivatives containing tertiary amino groups and quaternary ammonium groups.

16. Process for the preparation of mono- or poly-quaternary polysiloxanes according to any one of Claims 1 to 15, **characterized in that**, for the preparation of compounds carrying tertiary amino functions and quaternary ammonium groups and of the general structure
S-K-Q¹-K-S
di-secondary amines, preferably piperazine, are converted to compounds carrying tertiary amino structures with monofunctional, reactive, alkylating siloxane intermediates, in which the stoichiometry of the di-secondary amine to the reactive, alkylating siloxane is 1:2 and subsequently an alkylation, with epoxides in the presence of acids, alkyl halides or halocarboxylic acid esters, preferably with halocarboxylic acid esters of the alkylene oxides, takes place, in which the molar ratio of the tertiary amino groups to the alkylating agents is expediently 100:1 to 1:1.

17. Process for the preparation of polysiloxane derivatives according to any one of Claims 1 to 16, **characterized in that**, for the preparation of compounds containing quaternary ammonium groups and tertiary amino functions and of the general structure
S-K-Q¹-K-S
secondary amines, preferably dimethylamine, or secondary-tertiary diamines, preferably N-methylpiperazine, are converted into compounds carrying tertiary or di-tertiary aminosiloxane structures with monofunctional, reactive, alkylating siloxane intermediates, preferably epoxy derivatives, where the stoichiometry of the secondary amine or of the secondary-tertiary diamine to the monofunctional, reactive, alkylating siloxane is expediently about 1:1, and subsequently the formed tertiary or di-tertiary aminosiloxane structures are reacted with a difunctional alkylating agent preferably in the molar ratio 2:1 to give siloxane derivatives containing quaternary ammonium groups or quaternary ammonium groups and at the same time tertiary amino structures.

18. Process for the preparation of siloxane derivatives according to any one of Claims 8 to 17, **characterized in that** the halocarboxylic acid esters based on low-molecular-weight, oligomeric and polymeric alkylene oxides of the general composition
HO [CH₂CH₂O]_{q}- [CH₂CH(CH₃)O]ᵣR⁴
in which q, r and R⁴ have the meanings according to Claim 1, and preferably monosubstituted derivatives from the group consisting of diethylene glycol, triethylene glycol, tetraethylene glycol, or oligoethylene glycols with molecular weights of from 300 to 1000 g/mol, and dipropylene glycol are used.

19. Process for the preparation of siloxane derivatives according to one of Claims 8 to 18, **characterized in that** the halocarboxylic acid esters based on low-molecular-weight, oligomeric and polymeric alkylene oxides used are preferably those from the group of oligoethylene glycols with molecular weights of 400, 600 and 800 g/mol.

20. Use of mono- or poly-quaternary polysiloxanes in which two siloxane units are joined together via amino or ammonium units, according to Claims 1 to 7 or which are obtainable by one of Claims 8 to 19, in cosmetic formulations for skincare and haircare, in polishes for the treatment and finishing of hard surfaces, in formulations for the drying of automobiles and other hard surfaces after machine washing, for the finishing of textiles and textile fibres, as separate softeners after the washing of textiles with nonionogenic or anionic/nonionogenic detergent formulations, as softeners in formulations for textile washing based on nonionic or anionic/nonionic surfactants, where amino groups are used in the form of the amines or amine salts depending on the pH.

## Revendications

1. Polysiloxanes mono- ou polyquaternaires de la formule générale (I)
S-K-Q¹-K-S (I)
dans laquelle
S S 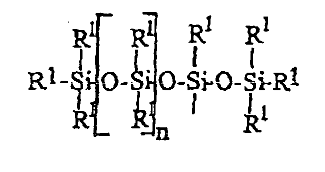
ou 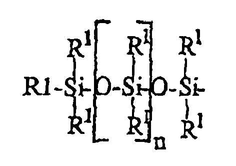
R¹ représente un alkyle en C₁ à C₂₂, un fluoro-alkyle en C₁ à C₂₂ ou un aryle,
n est compris entre 0 et 1000
Q¹ est une structure amine secondaire 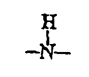 ou une structure amine tertiaire 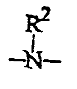 ou une structure ammonium quaternaire 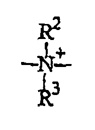 R² représente un radical hydrocarbure en C₁ à C₃₀ monovalent ou divalent, à chaîne droite, cyclique ou ramifiée, qui peut être interrompu par -O-, -NH-, -C(O)-, -C(S)-, et substitué par -OH, ou représente une simple liaison au radical K,
R³ représente un radical hydrocarbure en C₁ à C₃₀ monovalent, à chaîne droite, cyclique ou ramifiée, qui peut être interrompu par -O-, -NH-, -C(O)-, -C(S)-, et substitué par -OH, ou une structure -A-E-, dans laquelle
A représente -CH₂C(O)O-, -CH₂CH₂C(O)O- ou -CH₂CH₂CH₂C(O)O- et
E représente une unité poly(oxyde d'alkylène) de la structure
-[CH₂CH₂O]_{q}-[CH₂CH₂(CH₃)O]ᵣ-R⁴
q est compris entre 1 et 200
r est compris entre 0 et 200
R⁴ correspond à H, à un radical hydrocarbure en C₁ à C₂₀ à chaîne droite, cyclique ou ramifiée, qui peut être interrompu par -O- ou -C(O)-, et substitué par -OH, et qui peut être acétylénique, oléfinique ou aromatique,
sachant que, si une pluralité de radicaux R³ est présente dans la molécule, ceux-ci peuvent être identiques ou différents, ainsi que
K représente un radical hydrocarbure divalent ou trivalent en C₁ à C40 à chaîne droite, cyclique ou ramifiée, qui peut être interrompu par -O-, -NH-, -NR¹-, 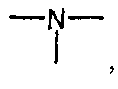 -C(O)-, - C(S)-, et substitué par -OH, ou qui contient une unité Q², avec
Q² est une structure amine secondaire 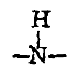 ou une structure amine tertiaire 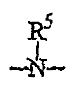 ou une structure ammonium quaternaire 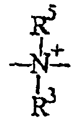 R⁵ étant un radical hydrocarbure en C₁ à C₂₀ monovalent ou divalent, à chaîne droite, cyclique ou ramifiée, qui peut être interrompu par -O-, -NH-, -C(O)-, -C(S)-, et substitué par -OH, sachant que la valence libre du radical divalent R⁵ peut se lier à Q¹,
et que, quand une pluralité de radicaux K est présente dans les polysiloxanes, ceux-ci peuvent être identiques ou différents les uns des autres,
à l'exception du composé de la formule

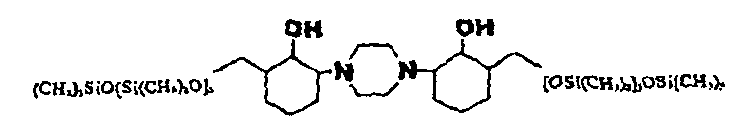

2. Polysiloxanes mono- ou polyquaternaires selon la revendication 1, **caractérisés en ce que** n est de 0 à 100, de préférence de 0 à 80, et de façon particulièrement préférée de 10 à 80.

3. Polysiloxanes mono- ou polyquaternaires selon l'une des revendications précédentes, **caractérisés en ce que** q est de 1 à 50.

4. Polysiloxanes mono- ou polyquaternaires selon l'une des revendications précédentes, **caractérisés en ce que** r est de 0 à 100 et de préférence de 0 à 50.

5. Polysiloxanes mono- ou polyquaternaires selon l'une des revendications précédentes, **caractérisés en ce que** R² et R³ sont ―CH₃, -CH₂CH₃, -(CH₂)₂CH₃, -(CH₂)₃CH₃, - (CH₂)₅CH₃, -CH₂CH₂OH, 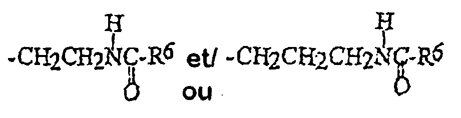 où R⁶ est un radical hydrocarbure en C₁ à C₁₈ à chaîne droite, cyclique ou ramifiée, qui peut être interrompu par -O-, -NH-, -C(O)-, -C(S)-, et substitué par-OH.

6. Polysiloxanes mono- ou polyquaternaires selon l'une des revendications précédentes, **caractérisés en ce que** R³ est -CH₃, -CH₂CH₃, -(CH₂)₂CH₃, -(CH₂)₃CH₃, - (CH₂)₅CH₃, -CH₂CH₂OH, 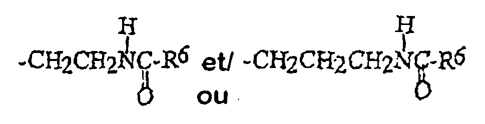 où R⁶ est un radical hydrocarbure en C₁ à C₁₈ à chaîne droite, cyclique ou ramifiée, qui peut être interrompu par -O-, -NH-, -C(O)-, -C(S)-, et substitué par ―OH.

7. Polysiloxanes mono- ou polyquaternaires selon l'une des revendications précédentes, **caractérisés en ce que** K représente un radical hydrocarbure divalent ou trivalent en C₁ à C₃₀ à chaîne droite, cyclique ou ramifiée, qui peut être interrompu par -O-, -NH-, - NR¹-, 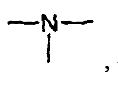 -C(O)-, -C(S)-, et substitué par -OH, et/ou qui contient une unité Q².

8. Procédé de préparation de polysiloxanes mono- ou polyquaternaires selon l'une quelconque des revendications 1 à 7, **caractérisé en ce que**, pour la préparation des composés contenant des groupes ammonium quaternaire de la structure générale
S-K-Q¹-K-S
dans laquelle Q¹ est 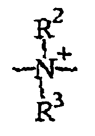 des dérivés monofonctionnels du siloxane contenant des fonctions amines tertiaires sont alkylés avec des dérivés monofonctionnels réactifs du siloxane, qui sont synthétisés par hydrosilylation, par exemple d'alcènes halogénés, d'esters d'acides carboxyliques halogénés et d'alcènes à fonction époxyde, par des composés SiH monofonctionnels des structures générales 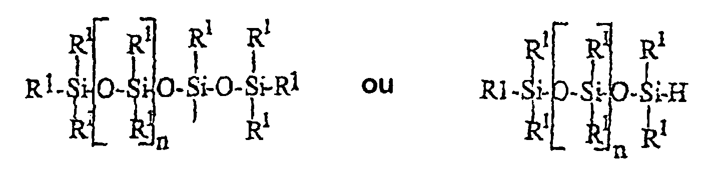 dans lesquelles le rapport molaire des fonctions amines tertiaires aux groupes alkylants réactifs est de manière appropriée de 100 : 1 à 1 : 1.

9. Procédé de préparation de polysiloxanes mono- ou polyquaternaires selon la revendication 8, **caractérisé en ce que**, comme alcènes halogénés sont utilisés de préférence le chlorure d'allyle et le bromure d'allyle.

10. Procédé de préparation de polysiloxanes mono- ou polyquaternaires selon la revendication 8, **caractérisé en ce que**, comme esters d'acides carboxyliques halogénés insaturés, on utilise de préférence des composés parmi le groupe constitué de l'ester allylique de l'acide chloracétique, de l'ester propargylique de l'acide chloracétique, de l'ester allylique de l'acide 3-chloropropionique et de l'ester propargylique de l'acide 3-chloropropionique.

11. Procédé de préparation de polysiloxanes mono- ou polyquaternaires selon la revendication 8, **caractérisé en ce que**, comme alcènes à fonction époxyde, on utilise de préférence de l'oxyde de vinylcyclohexène et de l'éther allylglycidique.

12. Procédé de préparation de polysiloxanes mono- ou polyquaternaires selon l'une quelconque des revendications 1 à 7, **caractérisé en ce que**, pour la préparation de composés portant des fonction amines tertiaires de la formule générale
S-K-Q¹-K-S
dans laquelle
Q¹ est 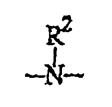 et K et S ont la signification conformément à la revendication 1, des structures insaturées portant des fonctions amines sont liées directement par hydrosilylation au Si-H-siloxane monofonctionnel et sont ensuite converties avec des produits intermédiaires monofonctionnels réactifs alkylants du siloxane en des composés portant des structures aminées tertiaires, dans lesquelles la stoechiométrie de l'amine secondaire au siloxane réactif alkylant est de manière appropriée de 1 : 1.

13. Procédé de préparation de polysiloxanes mono- ou polyquaternaires selon la revendication 12, **caractérisé en ce que**, comme structures insaturées portant des fonctions amines secondaires, on utilise de préférence de la N-méthylallylamine ou le composé CH₂=CHCH₂OCH₂CH(OH)CH₂NHCH₃.

14. Procédé de préparation de polysiloxanes mono- ou polyquaternaires selon l'une quelconque des revendications 1 à 7, **caractérisé en ce que**, pour la préparation de composés portant des fonctions amines tertiaires de la structure générale
S-K-Q¹-K-S
dans laquelle Q¹ est 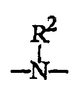 et K et S ont la signification conformément à la revendication 1, des di(amines secondaires), de préférence la pipérazine, sont converties avec des produits intermédiaires monofonctionnels réactifs alkylants du siloxane en des composés portant des structures amines tertiaires, sachant que la stoechiométrie de la di-(amine secondaire) au siloxane réactif alkylant est de manière appropriée d'environ 1 : 2.

15. Procédé de préparation de polysiloxanes mono- ou polyquaternaires selon l'une quelconque des revendications 1 à 7, **caractérisé en ce que**, pour la préparation de quantités équimolaires de composés contenant des fonctions amines tertiaires et des groupes ammonium quaternaires, de 1a structure générale
S-K-Q¹-K-S
des diamines secondaires-tertiaires, de préférence la N-méthylpipérazine, sont converties avec des produits intermédiaires monofonctionnels réactifs alkylants du siloxane, de préférence des dérivés époxydés, en des composés portant des structures d'aminosiloxanes di-tertiaires, sachant que la stoechiométrie de la diamine secondaire-tertiaire au siloxane monofonctionnel réactif alkylant est de manière appropriée environ 1 : 1, et qu'ensuite les structures d'aminosiloxane di-tertiaire sont converties avec une mole d'un composé siloxane monofonctionnel réactif alkylant en les dérivés du siloxane contenant des groupes amines tertiaires et des groupes ammoniums quaternaires.

16. Procédé de préparation de polysiloxanes mono- ou polyquaternaires selon l'une quelconque des revendications 1 à 15, **caractérisé en ce que**, pour la préparation de composés portant des fonctions amines tertiaires et des groupes ammoniums quaternaires de la structure générale
S-K-Q¹-K-S
des di(amines secondaires), de préférence la pipérazine, sont converties avec des produits intermédiaires monofonctionnels réactifs alkylants du siloxane en des composés portant des structures amines tertiaires, sachant que la stoechiométrie de la di(amine secondaire) au siloxane réactif alkylant est de 1 : 2 et **en ce qu'**il s'effectue ensuite une alkylation avec des époxydes en présence d'acides, d'halogénures d'alkyles ou d'esters d'acides carboxyliques halogénés, de préférence avec des esters d'acides carboxyliques halogénés des oxydes d'alkylène, dans laquelle le rapport molaire des groupes amines tertiaires aux réactifs d'alkylation est compris de manière appropriée entre 100 : 1 et 1 : 1.

17. Procédé de préparation de polysiloxanes mono- ou polyquaternaires selon l'une quelconque des revendications 1 à 16, **caractérisé en ce que**, pour la préparation de composés portant des groupes ammoniums quaternaires et des fonctions amines tertiaires de la structure générale
S-K-Q¹-K-S
des amines secondaires, de préférence la diméthylamine, ou des diamines secondaires-tertiaires, de préférence la N-méthylpipérazine, sont converties avec des produits intermédiaires monofonctionnels réactifs alkylants du siloxane, de préférence des dérivés époxydés, en des composés portant des structures d'aminosiloxane tertiaires ou ditertiaires, dans lesquels la stoechiométrie de l'amine secondaire ou de la diamine secondaire-tertiaire au siloxane monofonctionnel réactif alkylant est de manière appropriée d'environ 1 : 1, et **en ce qu'**ensuite les structures d'aminosiloxanes tertiaires, respectivement di-tertiaires formées sont converties avec un agent alkylant difonctionnel, de préférence dans le rapport molaire de 2 : 1, en des dérivés du siloxane contenant des groupes ammoniums quaternaires ou des groupes ammoniums quaternaires et en même temps des structures amines tertiaires.

18. Procédé de préparation de dérivés du siloxane selon l'une quelconque des revendications 8 à 17, **caractérisé en ce que** les esters d'acides carboxyliques halogénés sont utilisés sur des oxydes d'alkylène de bas poids moléculaire, oligomères et polymères de la composition générale
HO[CH₂CH₂O]_{q}-[CH₂CH(CH₃)O]ᵣR⁴
dans laquelle q, r et R⁴ ont la signification conformément à la revendication 1, et de préférence sur des dérivés monosubstitués du groupe consistant en le diéthylèneglycol, le triéthylèneglycol, le tétraéthylèneglycol, ou des oligoéthylèneglycols avec des poids moléculaires de 300 à 1000 g/mol, et le dipropylèneglycol.

19. Procédé de préparation de polysiloxanes mono- ou polyquaternaires selon l'une quelconque des revendications 8 à 18, **caractérisé en ce que**, comme esters d'acides carboxyliques halogénés sur des oxydes d'alkylène de bas poids moléculaire, oligomères et polymères, sont utilisés de préférence des composés du groupe des oligoéthylèneglycols avec des poids moléculaires de 400, 600 et 800 g/mol.

20. Utilisation de polysiloxanes mono- ou polyquaternaires, dans lesquels deux unités de siloxane sont reliées entre elles par des unités amines ou ammonium, selon les revendications 1 à 7, ou qui peuvent être obtenus selon l'une des revendications 8 à 19, dans des formulations cosmétiques pour les soins de la peau et des cheveux, dans des vernis pour le traitement et le finissage de surfaces dures, dans des formulation pour le séchage des automobiles et d'autres surfaces dures après un lavage mécanique, pour l'apprêt de textiles et de fibres textiles, comme adoucissant séparé après le lavage de textiles avec des formulations de détergents non ionogènes ou anioniques/non ionogènes, comme adoucissants dans des formulations se basant sur des tensioactifs non ioniques ou anioniques/non ioniques pour le lavage des textiles, sachant qu'en fonction du pH, des groupes amines sont utilisés sous forme des amines ou des sels d'amines.
